# EUROPEAN PATENT APPLICATION

(11) **EP 3 415 010 A1**
(43) Date of publication of application: **19.12.2018**
(21) Application number: 17175851.9
(22) Date of filing: 13.06.2017
(51) Int. Cl.: A01N 63/02, A01N 63/04, A01N 25/00, A01P 7/04, C12N 15/00, A61K 38/17, A61K 39/395, C07K 16/18

(54) **INSECT-CONTROLLING POLYPEPTIDES AND METHODS**

(71) Applicant: AGROSAVFE NV, 9052 Zwijnaarde (BE)
(72) Inventor: Dedecker, Maarten, 8310 Assebroek (BE); De Keyser, Julie, 9820 Merelbeke (BE); Peferoen, Marnix, 9881 Bellem (BE)
(74) Representative: De Clercq & Partners

(57) **Abstract**

The present invention relates to the control of pest insects. More specifically the invention relates to the identification, isolation and characterization of polypeptides for use in the control of pest insects. The invention thus provides insect-controlling peptides. Also provided are nucleic acids encoding such insect-controlling polypeptides and host cells and plants expressing such polypeptides. The present invention further provides compositions comprising polypeptides which are useful to combat crop pest insects in agriculture, insects which are pests for humans and animals, and house-hold pest insects. Also provided are methods for combating such pest insects.

## Description

### Field of the invention

The present invention relates to the control of pest insects. More specifically the invention relates to the identification, isolation and characterization of polypeptides for use in the control of pest insects. The present invention further provides compositions comprising polypeptides which are useful to combat crop pest insects in agriculture and house-hold pest insects.

### Background

The global production of food by modern agriculture is heavily challenged by pest insects. Farmers rely on insecticides to reduce insect damage, yet available commercial options for safe and functional insecticides are diminishing through the removal of hazardous chemicals from the marketplace and the emergence of insect strains that are resistant to all major classes of chemical and biological insecticides. New non-chemical insecticides are necessary to protect plants and crops in a sustainable and environment-friendly way.

Insecticidal peptides are peptides that are toxic to insects. They may have the ability to inhibit development and growth of the insect, impair its movement, reduce fecundity or render sterile, or even kill an insect. Insecticidal peptides are delivered to the insect by spreading the toxin into the habitat or environment of the insect by spraying or other means, so that the insect comes into contact with the peptide. Insecticidal peptides may be delivered as such or as microorganisms producing the peptides. Alternatively, insecticidal peptides are expressed in the organisms on which the pest insects live or feed.

Toxins derived from *Bacillus thuringiensis* (Bt) are insecticidal peptides well known in the art. Following ingestion by the insect the crystal is processed by gut proteases to release the mature active toxin (Cry proteins or δ-endotoxins). The mature toxin interacts with specific surface receptors of midgut cells and forms pores in the membrane, followed by disruption of ionic balance and death of the cells, and eventually death of the insect. Many Cry proteins are limited in their usefulness as insecticides because their host range is rather narrow. Furthermore, an increasing number of insect species have developed resistance to Bt toxins (Carrière et al., 2016).

Several attempts have been taken to increase the host range and/or toxicity of toxins. In EP 0 340 948 A1 is disclosed a hybrid toxin comprising the gut-binding portion of a Bt toxin and an ADP-ribosylating enzyme as a cytotoxic agent. WO 1991/017254 A1 discloses hybrid toxins comprising Cry protein coupled to additional gut-binding domains such as gp64 of Autographa californica Multiple Nuclear Polyhedrosis (AcMNP) Virus. WO 2000/066755 A2 describes hybrid toxins comprising a gut-binding domain such as a lectin domain fused to a toxic domain derived from a Cry protein. In US 2008/0300210 A1 is disclosed a hybrid toxin comprising a midgut receptor-binding domain of an envelope/membrane glycoprotein from a virus capable of infecting the target pest insect, and an active toxic fragment of a Bt toxin. In US 2013/0097729 A1 is described a chimeric insecticidal toxin comprising a Cry toxin and one or more peptides binding to the gut of sap-sucking insects. US 2015/0337294 A1 describes how recombinant plant beta-barrel binding proteins and alpha-helical coiled-coil polypeptides can be used to direct a fused second polypeptide, such as a toxin (e.g. a Cry protein), to a target molecule such as molecule found on the surface of an insect cell. Also disclosed is how plant-derived beta-barrel binding proteins or alpha-helical coiled-coil polypeptides that bind a particular receptor protein or glycoprotein in the gut of an a target insect pest of a plant can be coupled to a segment of an insecticidal protein that does not itself convey insecticidal properties, but which, when linked to such beta-barrel binding proteins or alpha-helical coiled-coil polypeptides and allowed to bind to such receptor, bring about an insecticidal effect upon such insect pest.

Antibodies against insect gut proteins have been described previously. EP 0 526 397 A1 discloses antibodies against Bt toxin-binding receptors in the insect gut. The antibodies are useful in test kits, to identify δ-endotoxins, to determine changes in the number of Bt toxin-binding receptors, to determine availability and accessibility of Bt toxin-binding receptors in the insect gut, and to analyze resistance against Bt toxin. No intrinsic insecticidal activity of the antibodies is disclosed. In WO 2001/034807 A2 antibodies against a receptor for Bt toxin are described. The antibodies are used in immunodetection and Western blot analysis. No intrinsic insecticidal activity of the antibodies is disclosed. WO 2002/011757 A2 describes anti-mosquito midgut antibodies able to provide an effective barrier to malarial ookinete penetration of, and encystment within, the wall of the midgut, thereby inhibiting development of the malarial parasite and reducing transmission of the disease. No intrinsic insecticidal activity of the antibodies is disclosed.

Antibodies have also been used to increase the host range and/or toxicity of Bt toxins and other toxins. In WO 1996/000783 A1 antibodies which bind to brush border membrane vesicles (BBMV) of insect gut are disclosed. The antibodies are used in constructing hybrid toxins, more specifically hybrid δ-endotoxins. WO 1999/002991 A1 describes antibodies and phages displaying single chain antibody fragments that bind to the cells of the microvilli of the midgut of fire ants. The antibodies and phages are conjugated to toxins, e.g. gelonin, for targeted delivery of the toxin. In WO 1994/004678 A1 variable domains of camelid heavy chain immunoglobulins, VHHs, are disclosed. WO 1994/004678 A1 further suggests a potential advantageous combination between a VHH recognizing an insect gut antigen and a toxin specific for insects to increase the specificity or host range of the toxin.

Several documents disclose immunization of animal hosts with antigens from blood and tissue feeding insects. Upon feeding and ingestion, the resulting antisera and antibodies often reduce fecundity, delay insect development, inhibit insect metabolism, reduce feeding on animal hosts, or increase mortality. WO 1992/003156 A1 discloses vaccine compositions comprising flea membrane extract, such as midgut membrane extract, effective to confer resistance against flea infestations, and polyclonal antibodies specifically immunoreactive with said compositions. Upon immunization of sheep with the vaccine compositions, 12% fewer live fleas were recovered as compared to the number of live fleas recovered from non-immunized sheep. US patent 5,418,137 discloses flea midgut membrane binding sites, more in particular the α-subunit of (Na⁺/K⁺)ATPase, as potential targets for flea control agents. Antisera and monoclonal antibodies are also disclosed as a tool in competition analysis of potential flea control agents. The flea midgut membrane binding sites are speculated to be useful for vaccine development, whereby antibodies would be generated in the immunized animal that interfere with the metabolism of the fleas. WO 1993/011790 A1 discloses vaccines comprising antigens derived from the supernatant fractions of midgut extracts of fleas. Feeding of ammonium sulfate-precipitated immunoglobulins from polyclonal antisera in blood is shown to result in increased flea mortality. Alger and Cabrera (Alger and Cabrera, 1972) describe how feeding of *Anopheles stephensi* mosquitos on rabbits immunized with whole midgut extracts resulted in a higher death rate. Sutherland and Ewen (Sutherland and Ewen, 1974) showed that female *Aedes aegypti* mosquitos feeding on rabbits immunized with whole mosquito extracts had a decreased fecundity. Schlein and Lewis (Schlein and Lewis, 1976) disclose that *Stomoxys calcitrans,* the stable fly, fed on rabbits which have been immunized with fly tissues, especially flight muscle proteins, show increased mortality. Casu *et al.* (Casu et al., 1997) show that antisera from sheep immunized with the peritrophic membrane protein peritrophin 95 from the fly *Lucilia cuprina* inhibit the growth of the flies when fed to them. Ghosh and Mukhopadhyay (Ghosh and Mukhopadhyay, 1998) describe that upon repeated bites of the sand fly *Phlebotomus argentipes,* hamsters develop antibodies against salivary gland proteins of the flies, which when fed to the flies lead to increased mortality. In the case of animal host resistance after vaccinations, it remains to be determined which effector components of the host immune system, i.e. antibodies, complement or cells of the immune system, are active against the insect.

US Patent No. 5,665,595 discloses monoclonal antibodies binding to an antigenic determinant on the midgut intestinal membrane tissue of Lepidopteran and Coleopteran species. The recognized antigenic determinants are not specified. It is speculated that the antibodies could be toxic, injuring or repelling to the insect, either directly or through the toxic activity of a conjugated insect growth inhibitor. No insecticidal activity is demonstrated. In WO 1999/067373 A2 antisera against the peritrophic membrane mucin proteins IIM14 and IIM22 of *Trichoplusia ni,* a lepidopteran pest, are disclosed. Purified polyclonal IgG antibodies can be used in Western blot and immunodetection analysis of IIMs. Feeding of the IgG to larvae results in delayed development. Meyers *et al.* (Meyers et al., 2015) show that feeding of polyclonal antibodies against neuronal glutamate-gated chloride channel to *Anopheles gambiae* results in increased mortality of the mosquitos.

EP 3 048 171 A1 discloses insecticidal activity of an anti-idiotype single-chain antibody selected against polyclonal anti-Cry1C. The recognized antigenic determinant is not specified.

In the light of the above discussed prior art, there remains a need for stable, effective, specific, and defined insect-controlling polypeptides which can be applied to objects, crops animals, or humans, or expressed by microbial organisms or crops and which offer a save and environmentally friendly alternative to chemical insecticides.

### Summary of the invention

The present inventors have successfully developed insect-controlling polypeptides with surprisingly high specificity, affinity and potency against pest insects, in particular plant insect pests, such as but not limited to plant chewing insect pests, plant sap-sucking insect pests and plant piercing insect pests, insect pests to animals and humans, or household insect pests. Moreover, it is shown that these polypeptides retain their integrity, stability and activity in agrochemical, pharmaceutical, or other compositions (as further defined herein) and that efficacious insect pest can surprisingly be achieved by applying agrochemical, pharmaceutical, or other compositions, comprising the polypeptides as disclosed in the present application, to crops, animals, humans, or objects.

The efficacy and potency of the polypeptides as disclosed herein suggests a potential for either a lower treatment dosage and/or a more effective treatment at the same dose. This can imply a reduction of unwanted side-effects and reduced toxicity, e.g. to non-target organisms or treated plants, animals, or humans. Moreover, this allows the application of lower amounts of the polypeptides or compositions disclosed herein.

More particularly, the present inventors have found that targeting a molecular structure of a target insect pest with the polypeptides envisaged herein allows for efficient control of that target insect pest when applied directly to the pest or on a plant or on one or more parts of a plant, on animals, on humans, or on objects.

In particular, the present inventors have developed polypeptides that are capable of controlling insect pests. Therefore, the present invention demonstrates for the first time that biological molecules, such as polypeptides, can be used to effectively protect or treat a plant, animal, human, or object, from being damaged or harmed in any way by or suffering from a biological interaction between the plant, animal, human, or object, and an insect pest such as for instance through a insect pest infestation.

In a first aspect, the present invention provides insect-controlling polypeptides comprising a binding domain capable of binding a binding site of an insect. In a preferred embodiment, the binding site is in the digestive tract of the insect. In a more preferred embodiment, the binding site is in the midgut and/or hindgut. of the insect. In particular embodiments, the polypeptides are capable of controlling said insect upon uptake by said insect. In a particular embodiment, the binding site is specific for the midgut. In a more particular embodiment, the binding domain is located on the columnar cells, goblet cells, regenerative cells, or endocrine cells of the midgut. In a particular embodiment, the binding site is located at the brush border of the midgut. In a more particular embodiment, the binding site is cell membrane associated. In a particular embodiment, the binding site is selected from the group consisting of CAATCH1, KAAT1, V-ATPase subunit a, V-ATPase subunit c, V-ATPase subunit e, IIM14, IIM21, ABCH1, SSK, MESH, K⁺/2H⁺ antiporter, Cl⁻ channel, and Cl⁻/HCO₃⁻ antiporter. In a preferred embodiment, the insect is of the order of *Lepidoptera.*

In a preferred embodiment, the binding domain is an antigen-binding domain. In a more preferred embodiment, the antigen-binding domain is selected from the group consisting of VHHs and alphabodies, and functional fragments thereof.
In a particular embodiment, the insect-controlling polypeptides further comprise at least one additional binding domain.
In a particular embodiment, the insect-controlling polypeptides further comprise a toxin moiety. In a more particular embodiment, the toxin moiety is a δ-endotoxin from *Bacillus thuringiensis* or a functional fragment thereof. In an even more particular embodiment, the toxin moiety is selected from the group consisting of Cry1, Cry2, and Cry15.
In a particular embodiment of the insect-controlling polypeptides, controlling said insect means to inhibit or reduce the ability of said insect to feed, grow, develop, survive, and/or reproduce, and/or to limit insect-related damage. In a particular embodiment of the insect-controlling polypeptides, controlling said insect is selected from the group consisting of killing said insect, decreasing said insect's survival and/or longevity, decreasing said insect's fecundity and/or fertility, decreasing or arresting said insect's feeding, decreasing or arresting said insect's growth, decreasing or arresting said insect's development, and deterring said insect.
In a particular embodiment, the insect-controlling polypeptides control said insect upon uptake by said insect. In a more particular embodiment, the insect-controlling polypeptides control said insect upon uptake by feeding.

In a second aspect, the present invention provides insect-controlling polypeptides as disclosed herein for use in medicine.

In a third aspect, the present invention provides nucleic acids comprising a polynucleotide encoding an insect-controlling polypeptide as disclosed herein.

In a fourth aspect, the present invention provides nucleic acid constructs comprising a nucleic acid comprising a polynucleotide encoding an insect-controlling polypeptide as disclosed herein. In a preferred embodiment, the nucleic acid constructs are an expression construct. In a more preferred embodiment, the nucleic acid comprised in the nucleic acid constructs is operably linked to at least one regulatory polynucleotide capable of driving expression of said nucleic acid. In an even more preferred embodiment, the regulatory polynucleotide is heterologous. In another preferred embodiment, the nucleic acid constructs are packaged in a viral particle, more preferably the viral particle is a bacteriophage or a baculovirus.

In a fifth aspect, the present invention provides host cells comprising an insect-controlling polypeptide as disclosed herein, a nucleic acid comprising a polynucleotide encoding an insect-controlling polypeptide as disclosed herein, or a nucleic acid construct comprising a nucleic acid comprising a polynucleotide encoding an insect-controlling polypeptide as disclosed herein. In one embodiment, the host cells are prokaryotic cells, more preferably bacterial cells. In another embodiment, the host cells are eukaryotic cells, more preferably fungal cells or plant cells or animal cells.

In a sixth aspect, the present invention provides compositions for preventing insect infestation and/or for controlling insects comprising at least one insect-controlling polypeptide as disclosed herein, a nucleic acid as disclosed herein, a nucleic acid construct as disclosed herein, or a host cell as disclosed herein. In a preferred embodiment, the compositions further comprise an agriculturally acceptable carrier or a pharmaceutically acceptable carrier. In a more preferred embodiment, the compositions further comprise at least one additional active ingredient. In an even more preferred embodiment, the additional active ingredient is selected from the group consisting of an insecticide, herbicide, fungicide, fertilizer, growth regulator, micro-nutrient, safener, pheromone, repellant, insect bait, and nucleic acid. In a preferred embodiment, the additional active ingredient is an insecticidal protein. In a more preferred embodiment, the insecticidal protein is selected from the group consisting of a *Bacillus thuringiensis* insecticidal protein, a *Xenorhabdus* insecticidal protein, a *Photorhabdus* insecticidal protein, a *Bacillus laterosporus* insecticidal protein, a *Bacillus bombysepticus* insecticidal protein, and a *Bacillus sphaericus* insecticidal protein. In a preferred embodiment, the additional active ingredient is an interfering RNA capable of targeting an insect gene. In a more preferred embodiment, the interfering RNA is comprised in a host cell. In a preferred embodiment, the compositions for preventing insect infestation and/or for controlling insects are a liquid, a spray, a mist, a coating, a gel, a paste, a powder, a bait, a trap, or a pellet.

In a seventh aspect, the present invention provides the use of insect-controlling polypeptides as disclosed herein, nucleic acids as disclosed herein, nucleic acid constructs as disclosed herein, host cells as disclosed herein, or compositions as disclosed herein, for preventing infestation by an insect or controlling an insect.
Also provided is the use of insect-controlling polypeptides as disclosed herein, nucleic acids as disclosed herein, nucleic acid constructs as disclosed herein, host cells as disclosed herein, or compositions as disclosed herein, as an insect-controlling agent.
Further provided is the use of insect-controlling polypeptides as disclosed herein, nucleic acids as disclosed herein, nucleic acid constructs as disclosed herein, host cells as disclosed herein, or compositions as disclosed herein, in medicine.

In an eight aspect, the present invention provides methods for controlling insects, comprising the step of contacting or feeding said insects with at least one insect-controlling polypeptide as disclosed herein, a nucleic acid as disclosed herein, a nucleic acid construct as disclosed herein, a host cell as disclosed herein, or a composition as disclosed herein.
The present invention further provides methods for preventing or treating infestation by an insect of a plant or plant part, comprising the step of applying to said plant or plant part or to the environment of said plant or plant part at least one insect-controlling polypeptide as disclosed herein, a nucleic acid as disclosed herein, a nucleic acid construct as disclosed herein, a host cell as disclosed herein, or a composition as disclosed herein.
Also provided are methods for preventing or treating infestation by an insect of a human or animal subject, comprising the step of applying to said human or animal subject or to the environment of said human or animal subject at least one insect-controlling polypeptide as disclosed herein, a nucleic acid as disclosed herein, a nucleic acid construct as disclosed herein, a host cell as disclosed herein, or a composition as disclosed herein.
The present invention further provides methods for preventing or treating infestation by an insect of an object, comprising the step of applying to said object or to the environment of said object at least one insect-controlling polypeptide as disclosed herein, a nucleic acid as disclosed herein, a nucleic acid construct as disclosed herein, a host cell as disclosed herein, or a composition as disclosed herein.

In a ninth aspect, the present invention provides plants or plant parts treated with and comprising at least one insect-controlling polypeptide as disclosed herein, a nucleic acid as disclosed herein, a nucleic acid construct as disclosed herein, a host cell as disclosed herein, or a composition as disclosed herein.

In a tenth aspect, the present invention provides transgenic plant cells transformed with a nucleic acid construct as disclosed herein. Also provided are transgenic plant cells comprising an insect-controlling polypeptide as disclosed herein, a nucleic acid as disclosed herein, or a nucleic acid construct as disclosed herein.
Further provided are transgenic plants transformed with a nucleic acid construct as disclosed herein. Also provided are transgenic plants comprising an insect-controlling polypeptide as disclosed herein, a nucleic acid as disclosed herein, or a nucleic acid construct as disclosed herein.
Also provided are seed of the transgenic plants as disclosed herein, wherein the seeds comprise an insect-controlling polypeptide as disclosed herein, a nucleic acid as disclosed herein, or a nucleic acid construct as disclosed herein.
Further provided are commodity product produced from a plant or plant part as disclosed herein, a transgenic plant cell as disclosed herein, a transgenic plant as disclosed herein, or the seed as disclosed herein, wherein said commodity product comprises an insect-controlling polypeptide as disclosed herein.

### Detailed description of the invention

The present invention will be described with respect to particular embodiments but the invention is not limited thereto.

Statements (features) and embodiments of the polypeptides, compositions and methods as disclosed herein are set here below. Each of the statements and embodiments as disclosed by the invention so defined may be combined with any other statement and/or embodiment unless clearly indicated to the contrary. In particular, any feature indicated as being preferred or advantageous may be combined with any other feature or features indicated as being preferred or advantageous.

Numbered statements as disclosed in the present application are:
1. An insect-controlling polypeptide comprising a binding domain capable of binding a binding site of an insect.
2. The insect-controlling polypeptide of statement 1, wherein said binding site is in the digestive tract of said insect.
3. The insect-controlling polypeptide of statement 2, wherein said binding site is in the midgut and/or hindgut of said insect.
4. The polypeptide of statement 3, wherein said binding site is specific for the midgut.
5. The insect-controlling polypeptide of statement 3 or 4, wherein said binding site is located on the columnar cells, goblet cells, regenerative cells, or endocrine cells of the midgut.
6. The insect-controlling polypeptide of any of statements 3-5, wherein said binding site is located at the brush border of the midgut of said insect.
7. The insect-controlling polypeptide of statement 5 or 6, wherein said binding-site is cell m em brane-associated
8. The polypeptide of any of statements 1 to 7, wherein said binding site is selected from the group consisting of CAATCH1, KAAT1, V-ATPase subunit a, V-ATPase subunit c, V-ATPase subunit e, IIM14, IIM21, ABCH1, SSK, MESH, K⁺/2H⁺ antiporter, Cl⁻ channel, and Cl⁻/HCO₃⁻ antiporter.
9. The insect-controlling polypeptide of any of statements 1-8, wherein said insect is of the order of *Lepidoptera.*
10. The insect-controlling polypeptide of any of statements 1-9, wherein said binding domain is an antigen-binding domain.
11. The insect-controlling polypeptide of statement 10, wherein said antigen-binding domain is selected from the group consisting of VHHs and alphabodies, and functional fragments thereof.
12. The insect-controlling polypeptide of any of statements 1-11, further comprising at least one additional binding domain.
13. The insect-controlling polypeptide of any of statements 1-12, further comprising a toxin moiety.
14. The insect-controlling polypeptide of statement 13, wherein said toxin moiety is a δ-endotoxin from *Bacillus thuringiensis* or a functional fragment thereof.
15. The insect-controlling polypeptide of statement 14, wherein said toxin moiety is selected from the group consisting of Cry1, Cry2, and Cry15.
16. The insect-controlling polypeptide of any of statements 1-15, wherein controlling said insect means to inhibit or reduce the ability of said insect to feed, grow, develop, survive, and/or reproduce, and/or to limit insect-related damage.
17. The insect-controlling polypeptide of any of statements 1-16, wherein controlling said insect is selected from the group consisting of killing said insect, decreasing said insect's survival and/or longevity, decreasing said insect's fecundity and/or fertility, decreasing or arresting said insect's feeding, decreasing or arresting said insect's growth, decreasing or arresting said insect's development, and deterring said insect.
18. The insect-controlling polypeptide of any of statements 1-17, wherein said insect-controlling polypeptide controls said insect upon uptake by said insect.
19. The polypeptide of statement 18, wherein uptake is by feeding.
20. The insect-controlling polypeptide of any of statements 1-19 for use in medicine.
21. A nucleic acid comprising a polynucleotide encoding an insect-controlling polypeptide according to any of statements 1-20.
22. A nucleic acid construct comprising the nucleic acid of statement 21.
23. The nucleic acid construct of statement 22, which is an expression construct.
24. The nucleic acid construct of statement 22 or 23, wherein said nucleic acid is operably linked to at least one regulatory polynucleotide capable of driving expression of said nucleic acid.
25. The nucleic acid construct of statement 24, wherein said regulatory polynucleotide is heterologous.
26. The nucleic acid construct of any of statements 22-25 which is packaged in a viral particle.
27. The nucleic acid construct of statement 26, wherein the viral particle is a bacteriophage or a baculovirus.
28. A host cell comprising the polypeptide of any of statements 1-20, the nucleic acid of statement 21, or the nucleic acid construct according to any of statements 22-25.
29. The host cell of statement 28 which is a prokaryotic or a eukaryotic cell.
30. The host cell of statement 28 which is a bacterial cell or a fungal cell.
31. The host cell of statement 28 which is a plant cell.
32. The host cell of statement 28 which is an animal cell.
33. A composition for preventing insect infestation and/or for controlling insects comprising at least one polypeptide according to any of statements 1-20, a nucleic acid of statement 21, a nucleic acid construct according to any of statements 22-27, or a host cell of any of statements 28-32.
34. The composition of statement 33, further comprising an agriculturally acceptable carrier or a pharmaceutically acceptable carrier.
35. The composition according to statement 33 or 34, further comprising at least one additional active ingredient.
36. The composition according to statement 35, wherein said active ingredient is selected from the group consisting of an insecticide, herbicide, fungicide, fertilizer, growth regulator, micro-nutrient, safener, pheromone, repellant, insect bait, and nucleic acid.
37. The composition according to statement 35 or 36, wherein said active ingredient is an interfering RNA capable of targeting an insect gene.
38. The composition of statement 37, wherein said interfering RNA is comprised in a host cell.
39. The composition according to statement 35 or 36, wherein said active ingredient is an insecticidal protein.
40. The composition of statement 39, wherein said insecticidal protein is selected from the group consisting of a *Bacillus thuringiensis* insecticidal protein, a *Xenorhabdus* insecticidal protein, a *Photorhabdus* insecticidal protein, a *Bacillus laterosporus* insecticidal protein, a *Bacillus bombysepticus* insecticidal protein, and a *Bacillus sphaericus* insecticidal protein.
41. The composition of any of statements 33-40, which is a liquid, a spray, a mist, a coating, a gel, a paste, a powder, a bait, a trap, or a pellet.
42. Use of a polypeptide according to any of statements 1-20, a nucleic acid of statement 21, a DNA construct according to any of statements 22-27, a host cell of any of statements 28-32, or a composition of any of statements 33-41 for preventing infestation by said insect or controlling said insect.
43. Use of a polypeptide according to any of statements 1-20, a nucleic acid of statement 21, a nucleic acid construct according to any of statements 22-27, a host cell of any of statements 28-32, or a composition of any of statements 33-41 as an insect-controlling agent.
44. Use of a polypeptide according to any of statements 1-20, a nucleic acid of statement 21, a nucleic acid construct according to any of statements 22-27, a host cell of any of statements 28-32, or a composition of any of statements 33-41 in medicine.
45. A method for controlling insects, comprising the step of contacting or feeding said insects with at least one polypeptide according to any of statements 1-20, a nucleic acid of statement 21, a nucleic acid construct according to any of statements 22-27, a host cell of any of statements 28-32, or a composition of any of statements 33-41.
46. A method for preventing or treating infestation by an insect of a plant or plant part, comprising the step of applying to said plant or plant part or to the environment of said plant or plant part at least one polypeptide according to any of statements 1-20, a nucleic acid of statement 21, a nucleic acid construct according to any of statements 22-27, a host cell of any of statements 28-32, or a composition of any of statements 33-41.
47. A method for preventing or treating infestation by an insect of a human or animal subject, comprising the step of applying to said human or animal subject or to the environment of said human or animal subject at least one polypeptide according to any of statements 1-20, a nucleic acid of statement 21, a nucleic acid construct according to any of statements 22-27, a host cell of any of statements 28-32, or a composition of any of statements 33-41.
48. A method for preventing or treating infestation by an insect of an object, comprising the step of applying to said object or to the environment of said object at least one polypeptide according to any of statements 1-20, a nucleic acid of statement 21, a nucleic acid construct according to any of statements 22-27, a host cell of any of statements 28-32, or a composition of any of statements 33-41.
49. A plant or plant part treated with and comprising at least one polypeptide according to any of statements 1-20, a nucleic acid of statement 21, a nucleic acid construct according to any of statements 22-27, a host cell of any of statements 28-32, or a composition of any of statements 33-41.
50. A transgenic plant cell transformed with a nucleic acid construct according to any of statements 22-27.
51. A transgenic plant cell comprising a polypeptide according to any of statements 1-20, a nucleic acid of statement 21, or a nucleic acid construct according to any of statements 22-27.
52. A transgenic plant transformed with the nucleic acid construct according to any of statements 22-27.
53. A transgenic plant comprising a polypeptide according to any of statements 1-20, a nucleic acid of statement 21, a nucleic acid construct according to any of statements 22-27.
54. A seed of the transgenic plant according to statement 52 or 53, wherein said seed comprises a polypeptide of any of statements 1-20, a nucleic acid of statement 21, a nucleic acid construct according to any of statements 22-27.
55. A commodity product produced from the plant or plant part according to statement 49, the transgenic plant cell of statement 50 or 51, the transgenic plant according to statement 52 or 53 or the seed of statement 54, wherein said commodity product comprises the polypeptide of any of statements 1-20.

### DEFINITIONS

Where the term "comprising" is used in the present description and statements, it does not exclude other elements or steps.

Where an indefinite or definite article is used when referring to a singular noun e.g. "a" or "an", "the", this includes a plural of that noun unless something else is specifically stated.
The term "about" as used herein when referring to a measurable value such as a parameter, an amount, a temporal duration, and the like, is meant to encompass variations of +/- 10% or less, preferably +/-5 % or less, more preferably +/- 1% or less, and still more preferably +/- 0.1 % or less of and from the specified value, insofar such variations are appropriate to perform in the disclosed invention. It is to be understood that the value to which the modifier 'about' refers is itself also specifically, and preferably, disclosed.

The following terms or definitions are provided solely to aid in the understanding of the invention. Unless specifically defined herein, all terms used herein have the same meaning as they would to one skilled in the art of the present invention. Practitioners are particularly directed to Sambrook et al., Molecular Cloning: A Laboratory Manual, 2nd ed., Cold Spring Harbor Press, Plainsview, New York (1989); and Ausubel et al., Current Protocols in Molecular Biology (Supplement 47), John Wiley & Sons, New York (1999), for definitions and terms of the art. The definitions provided herein should not be construed to have a scope less than understood by a person of ordinary skill in the art.

Unless indicated otherwise, all methods, steps, techniques and manipulations that are not specifically described in detail can be performed and have been performed in a manner known per se, as will be clear to the skilled person. Reference is for example again made to the standard handbooks, to the general background art referred to above and to the further references cited therein.

As used herein, the terms "determining", "measuring", "assessing", "monitoring" and "assaying" are used interchangeably and include both quantitative and qualitative determinations.

All documents cited in the present specification are hereby incorporated by reference in their entirety. Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

### [MACROMOLECULES, PROTEINS, NUCLEIC ACIDS, SEQUENCES]

As used herein, the term "macromolecule" refers to large molecules, preferentially of 1,000 or more atoms. Examples of macromolecules are biopolymers like nucleic acids, proteins, polysaccharides, and polyphenols, and large non-polymeric molecules such as lipids and macrocycles.

As used herein, the terms "protein", "peptide", "oligopeptide", and "polypeptide" are used interchangeably, and refer to an oligomeric or polymeric form of amino acids of any length or any configuration, linear, branched, or cyclic, which can include coded and non-coded amino acids, chemically or biochemically modified or derived amino acids, and polypeptides having modified peptide backbones. Polypeptides may have any two- and three-dimensional structure, and may perform any function, known or unknown.

As used herein, the terms "protein sequence", "peptide sequence", "polypeptide sequence", "amino acid sequence" are used interchangeably, and refer to the consecutive sequence or primary structure of amino acid residues of a protein or (poly)peptide.

As used herein, amino acid residues in a sequence will be indicated either by their full name or according to the standard three-letter or the IUPAC one-letter amino acid code.

As used herein, the terms "nucleic acid", "nucleic acid molecule", "polynucleotide", "polynucleic acid", and "oligonucleotide" are used interchangeably and refer to a polymeric form of nucleotides of any length or any configuration, linear, circular, or branched, the nucleotides being deoxyribonucleotides, ribonucleotides, or analogs and derivatives thereof. Polynucleotides may be partially or entirely single-stranded, double-stranded, triple-stranded, or quadruple-stranded. Polynucleotides may have any two- and three-dimensional structure, and may perform any function, known or unknown. Non-limiting examples of polynucleotides include chromosomes, genes, gene fragments, exons, introns, messenger RNAs (mRNA), antisense RNA (asRNA), non-coding RNAs, double-stranded RNAs (dsRNA), small interfering RNAs (siRNA), micro RNAs (miRNA), Piwi-interacting RNAs (piRNA), transfer RNAs (tRNA), ribosomal RNAs (rRNA), ribozymes, guide RNAs (gRNA), complementary DNAs (cDNA), aptamers, recombinant polynucleotides, synthetic polynucleotides, branched polynucleotides, plasmids, vectors, isolated DNAs of any sequence, control regions, promoters, terminators, enhancers, repressors, isolated RNAs of any sequence, nucleic acid probes, and primers. As used herein, the terms "nucleic acid sequence", "polynucleotide sequence", "DNA sequence", "RNA sequence" refer to the consecutive sequence of nucleotides of a (poly)nucleic acid, polynucleotide, DNA, or RNA molecule, respectively.

As used herein, nucleotides in a sequence will be indicated by their one-letter IUPAC nucleotide code. As used herein, the term "sequence homology" or "homology" denotes at least primary structural similarity between two or more macromolecules, particularly between two or more polypeptides or two or more polynucleotides, from same or different taxa, wherein the similarity is due to shared ancestry or convergent evolution. Hence, the term "homologues" denotes so-related macromolecules having at least primary structural similarity.

For comparing two or more polynucleotide sequences, the "(percentage of) sequence identity" between a first polynucleotide sequence and a second polynucleotide sequence may be calculated using methods known by the person skilled in the art, e.g. by optimally aligning the polynucleotide sequences and introducing gaps, if necessary, followed by dividing the number of nucleotides in the first polynucleotide sequence that are identical to the nucleotides at the corresponding positions in the second polynucleotide sequence in a comparison window by the number of positions in the comparison window (the window size), and multiplying by 100%. For comparing deoxyribonucleotides with ribonucleotides, (deoxy)thymidine is considered identical to uridine.

Optimal sequence alignment of two or more polynucleotide sequences over a comparison window can be obtained by using a known computer algorithm for sequence alignment such as NCBI Blast (Altschul et al., 1990). Another example of an algorithm that is suitable for polynucleotide sequence alignments is the CLUSTALW program (Thompson et al., 1994). CLUSTALW performs multiple pairwise comparisons between groups of sequences and assembles them into a multiple alignment based on homology.

As used herein, the term "sequence complementarity" or "complementarity" refers to sequence identity between a first polynucleotide and the complement of a second polynucleotide. For comparing deoxyribonucleotides with ribonucleotides, (deoxy)thymidine is considered identical to uridine.

For comparing two or more polypeptide sequences, the "(percentage of) sequence identity" between a first polypeptide sequence and a second polypeptide sequence may be calculated using methods known by the person skilled in the art, e.g. by optimally aligning the polypeptide sequences and introducing gaps, if necessary, followed by dividing the number of amino acids in the first polypeptide sequence that are identical to the amino acids at the corresponding positions in the second polypeptide sequence in a comparison window by the number of positions in the comparison window (the window size), and multiplying by 100%.

Optimal sequence alignment of two or more polypeptide sequences over a comparison window can be obtained by using a known computer algorithm for sequence alignment such as NCBI Blast (Altschul et al., 1990). Another example of an algorithm that is suitable for polypeptide sequence alignments is the CLUSTALW program (Thompson et al., 1994). CLUSTALW performs multiple pairwise comparisons between groups of sequences and assembles them into a multiple alignment based on homology. For amino acid alignments, the BLOSUM algorithm can be used as a protein weight matrix (Henikoff and Henikoff, 1992). In determining the "(percentage of) of sequence homology" between two amino acid sequences, the skilled person may take into account so-called "conservative" amino acid substitutions, which can generally be described as amino acid substitutions in which an amino acid residue is replaced with another amino acid residue of similar chemical structure and which has little or essentially no influence on the function, activity or other biological properties of the polypeptide. Conservative amino acid substitutions are counted as identities in order to calculate the percentage homology between two polypeptide sequences. Possible conservative amino acid substitutions will be clear to the person skilled in the art. A variety of known criteria indicate whether an amino acid that is substituted at a particular position in a peptide or polypeptide is conservative. For example, a conservative amino acid substitution is one in which an amino acid residue is replaced with an amino acid residue having a similar side chain. Similar amino acids may be included in the following categories: amino acids with basic side chains (e.g., lysine, arginine, histidine); amino acids with acidic side chains (e.g., aspartic acid, glutamic acid); amino acids with uncharged polar side chains (e.g., glycine, asparagine, glutamine, serine, threonine, tyrosine, cysteine, histidine); amino acids with nonpolar side chains (e.g., alanine, valine, leucine, isoleucine, proline, phenylalanine, methionine, tryptophan); amino acids with beta-branched side chains (e.g., threonine, valine, isoleucine), and amino acids with aromatic side chains (e.g., tyrosine, phenylalanine, tryptophan). Substitution of glutamine for glutamic acid or asparagine for aspartic acid may be considered a conservative substitution in that glutamine and asparagine are amide derivatives of glutamic acid and aspartic acid, respectively.

As used herein, "comparison window" makes reference to a contiguous and specified segment of an optimal alignment of polynucleotide or polypeptide sequences, wherein the sequences in the comparison window may comprise gaps for optimal alignment of the two sequences. The comparison window for determining (percentage of) sequence identity or homology may be as long as the longest of the aligned sequences including gaps, or as long as the shortest of the aligned sequences including gaps, or as long as the alignment including gaps in any of the sequences introduced to optimize the alignment. Comparison windows may be about 5000 positions long, or about 2000 positions, or about 1000 positions, or about 800 positions, or about 600 positions long, or about 500 positions long, or about 400 positions long, or about 300 positions long, or about 200 positions long, or about 100 positions long, or about 50 positions long, or about 40 positions long, or about 30 positions long, or about 20 positions long, or about 10 positions long. Comparison windows may be 25, 24, 23, 22, 21, 20, 19, or 18 positions long. Amino acid sequences or nucleic acid sequences are said to be "exactly the same" if they have 100% sequence identity over their entire length.

### [TARGET PESTS]

Disclosed herein are insect-controlling polypeptides. Thus in one embodiment, target pest organisms controlled are arthropods. "Arthropods" is used herein in the broad popular sense and includes all species of the phylum *Arthropoda* (Brands, S.J. (comp.) 1989-2005. Systema Naturae 2000. Amsterdam, The Netherlands. URL: sn2000.taxonomy.nl). It includes all the different phases of the life cycle of the arthropods, such as, but not limited to eggs, larvae, nymphs, pupae and adults. Preferably, "arthropods" includes insects, spiders, ticks, mites, chiggers, scrub-itch mites, feather mites, ear mites, scabies mites, Demodex, and scorpions.
"Arthropods" includes species of the subphylum *Arachnomorpha* and the subphylum *Mandibulata.* In a preferred embodiment, target pest organisms controlled belong to the subphylum *Mandibulata.* In a more preferred embodiment, target pest organisms controlled belong to the superclass *Panhexapoda.* In an even more preferred embodiment, target pest organisms controlled belong to the epiclass *Hexapoda.* In an even more preferred embodiment, target pest organisms controlled are insects, "insect" meaning any species belonging to the class *Insecta.*

Insects as used herein include but are not limited to:
- from the order *Lepidoptera,* for example: *Acleris* spp., *Adoxophyes* spp., *Agrotis* spp., *Alabama argillacea, Amyelois* spp., *Anticarsia gemmatalis, Archips* spp., *Argyrotaenia* spp., *Autographa* spp., *Busseola fusca, Cadra cautella, Carposina nipponensis, Chilo suppressalis, Chilo* spp., *Choristoneura conflictana, Choristoneura fumiferana, Choristoneura occidentalis, Choristoneura rosaceana, Choristoneura* spp., *Clysia ambiguella, Cnaphalocrocis* spp., *Cnephasia* spp., *Cochylis* spp., *Coleophora* spp., *Crocidolomia binotalis, Cryptophlebia leucotreta, Cydalima perspectalis, Cydia inopinata, Cydia* spp., *Diatraea* spp., *Diparopsis castanea, Earias* spp., *Ephestia* spp., *Eucosma* spp., *Eupoecilia ambiguella, Euproctis* spp., *Euxoa* spp., *Grapholita prunivora, Grapholita* spp., *Hedya nubiferanal, Helicoverpa armigera, Helicoverpa* zea, *Helicoverpa* spp., *Heliothis* spp., *Hellula undalis, Hyphantria cunea, Keiferia lycopersicella, Leucoptera scitella, Lithocolletis* spp., *Lobesia botrana, Lymantria* spp., *Lyonetia* spp., *Malacosoma* spp., *Mamestra brassicae, Manduca sexta, Numonia pyrivorella, Operophtera* spp., *Opogona sacchari, Ostrinia nubilalis, Pammene* spp., *Pandemis* spp., *Panolis flammea, Paysandisia archon, Pectinophora gossypiella, Phthorimaea operculella, Phyllonorycter* spp., *Pieris rapae, Pieris* spp., *Platynota rostrana, Plutella xylostella, Prays* spp., *Scirpophaga* spp., *Sesamia* spp., *Sesia* spp., *Sparganothis* spp., *Spodoptera dolichos, Spodoptera eridania, Spodoptera exigua, Spodoptera frugiperda, Spodoptera littoralis, Spodoptera litura, Spodoptera* spp., *Synanthedon* spp., *Tecia solanivora, Thaumatotibia leucotreta, Thaumetopoea processionea, Thaumetopoea* spp., *Tortrix* spp., *Trichoplusia ni,* and *Yponomeuta* spp.;
- from the order *Coleoptera,* for example, *Agrilus anxius, Agrilus planipennis, Agriotes* spp., *Anomala orientalis, Anoplophora chinensis, Anoplophora glabripennis, Anoplophora* spp., *Anthonomus bisignifer, Anthonomus eugenii, Anthonomus grandis, Anthonomus quadrigibbus, Anthonomus signatus, Anthonomus* spp., *Apriona* spp., *Arrhenodes minutus, Atomaria linearis, Chaetocnema tibialis, Conotrachelus nenuphar, Cosmopolites* spp., *Curculio* spp., *Dendroctonus micans, Dendrolimus sibiricus, Dermestes* spp., *Diabrotica virgifera virgifera, Diabrotica virgifera* zeae, *Diabrotica virgifera, Diabrotica balteata, Diabrotica barberi, Diabrotica undecimpunctata howardi, Diabrotica undecimpunctata tenella, Diabrotica undecimpunctata undecimpunctata, Diabrotica undecimpunctata, Diabrotica* spp., *Epilachna varivestis, Epilachna* spp., *Epitrix cucumeris, Eremnus cerealis, Eremnus* spp., *Gonipterus scutellatus, Ips amitinus, Ips cembrae, Ips duplicatus, Ips sexdentatus, Ips typographus, Ips* spp., *Leptinotarsa decemlineata, Leptinotarsa juncta, Leptinotarsa texana, Lissorhoptrus* spp., *Listronotus bonariensis, Melolontha* spp., *Monochamus* spp., *Naupactus leucoloma, Oryzaephilus* spp., *Otiorhynchus* spp., *Phlyctinus* spp., *Pissodes nemorensis, Pissodes strobi, Pissodes terminalis, Pissodes* spp., *Popilia japonica, Popilia* spp., *Premnotrypes* spp., *Pseudopityophthorus minutissimus, Pseudopityophthorus pruinosus, Psylliodes* spp., *Rhizopertha* spp., *Rhynchophorus ferrugineus, Rhynchophorus palmarum, Scarabaeidae* family spp., *Scolytidae* family spp., *Sitophilus* spp., *Sitotroga* spp., *Sternochetus mangiferae, Tenebrio* spp., *Tribolium castaneum, Tribolium* spp. and *Trogoderma* spp.;
- from the order *Orthoptera,* for example, *Gryllotalpa* spp., *Locusta* spp., and *Schistocerca* spp.;
- from the order *Blattaria,* from example, *Blatta* spp., *Blattella* spp., *Periplaneta* spp., and *Leucophaea maderae,*
- from the order *Isoptera,* for example, *Coptotermes* spp. and *Reticulitermes* spp.;
- from the order *Psocoptera,* for example, *Liposcelis spp*.;
- from the order *Phthiraptera,* suborder *Anoplura,* for example, *Haematopinus* spp., *Linognathus* spp., *and Pediculus* spp., and *Trichodectes* spp.;
- from the order *Phthiraptera,* suborder *Ischnocera,* for example, *Damalinia* spp.;
- from the order *Thysanoptera,* for example, *Frankliniella occidentalis, Frankliniella platensis, Frankliniella* spp., *Hercinothrips* spp., *Taeniothrips* spp., *Thrips palmi, Thrips tabaci, Scirtothrips aurantii, Scirtothrips citri, Scirtothrips dorsalis, and Scirtothrips* spp.;
- from the order *Hemiptera,* suborder *Heteroptera,* for example, *Cimex* spp., *Distantiella theobroma, Dysdercus* spp., *Euschistus* spp., *Eurygaster* spp., *Leptocorisa* spp., *Nezara* spp., *Piesma* spp., *Rhodnius* spp., *Sahlbergella singularis, Scotinophara* spp., *Triatoma* spp., *Miridae* family spp. *such* as *Lygus hesperus and Lygus lineoloris, Lygaeidae* family spp. *such* as *Blissus leucopterus, and Pentatomidae* family spp.;
- from the order *Hemiptera,* suborder *Sternorrhyncha,* for example, *Aleurocanthus spiniferus, Aleurocanthus woglumi, Aleurocanthus* spp., *Aleurothrixus floccosus, Aleyrodes brassicae, Aonidella citrina, Aonidiella* spp., *Aphididae* family spp., *Acyrthosiphon* spp., *Aphis fabae, Aphis glycines, Aphis gossypii, Aphis* spp., *Aspidiotus* spp., *Bemisia tabaci, Ceroplastes* spp., *Chrysomphalus aonidium, Chrysomphalus dictyospermi, Coccus hesperidum, Daktulosphaira vitifoliae, Diaphorina citri, Eriosoma larigerum, Gascardia* spp., *Lacanium corni, Lepidosaphes* spp., *Lopholeucaspis japonica, Macrosiphus* spp., *Margarodes prieskaensis, Margarodes vitis, Margarodes vredendalensis, Myzus persicae, Myzus* spp., *Parasaissetia nigra, Pemphigus* spp., *Phylloxera* spp., *Planococcus* spp., *Pseudaulacaspis* spp., *Pseudococcus* spp., *Psylla* spp., *Pulvinaria aethiopica, Quadraspidiotus* spp., *Rhopalosiphum* spp., *Ripersiella hibisci, Saissetia* spp., *Schizaphis* spp., *Sitobion* spp., *Toxoptera citricida, Trialeurodes vaporariorum, Trioza erytreae,* and *Unaspis citri;*
- from the order *Hemiptera,* suborder *Auchenorrhyncha,* for example, *Circulifer haematoceps, Circulifer tenellus, Draeculacephala minerva, Empoasca* spp., *Erythroneura* spp., *Graphocephala atropunctata, Hishimonus phycitis, Myndus crudus, Laodelphax* spp., *Nephotettix* spp., *Nilaparvata* spp., *Scaphoideus luteolus, Scaphoideus* spp., *and Xyphon fulgida;*
- from the order *Hymenoptera,* for example, *Acromyrmex, Atta* spp., *Cephus* spp., *Diprionidae* family spp. *such* as *Diprion* spp. *and Gilpinia polytoma, Hoplocampa* spp., *Lasius* spp., *Monomorium pharaonis, Neodiprion* spp., *Formicidae* family spp. *such* as *Solenopsis* spp., and Vespa spp.;
- from the order *Diptera,* for example, *Aedes albopictus, Aedes cinereus, Aedes polynesiensis, Aedes* spp., *Amauromyza maculosa, Anastrepha fraterculus, Anastrepha ludens, Anastrepha obliqua, Anastrepha suspensa, Anastrepha* spp., *Anopheles gambiae, Anopheles* spp., *Aschistonyx eppoi, Atherigona soccata, Bactrocera* spp., *Bibio hortulanus, Calliphora erythrocephala, Cephalcia lariciphila, Ceratitis rosa, Ceratitis* spp., *Chrysomyia* spp., *Culex* spp., *Cuterebra* spp., *Dacus* spp., *Drosophila melanogaster, Dryocosmus kuriphilus, Euphranta canadensis, Euphranta japonica, Fannia* spp., *Gastrophilus* spp., *Gilpinia hercyniae, Glossina* spp., *Hypoderma* spp., *Hippobosca* spp., *Liriomyza bryoniae, Liriomyza huidobrensis, Liriomyza sativae, Liriomyza trifolii, Liriomyza* spp., *Lucilia* spp., *Melanagromyza* spp., *Musca* spp., *Oestrus* spp., *Orseolia* spp., *Oscinella frit, Pardalaspis cyanescens, Pardalaspis quinaria, Pegomyia hyoscyami, Phorbia* spp., *Rhagoletis pomonella, Rhagoletis* spp., *Sciara* spp., *Stomoxys* spp., *Tabanus* spp., *and Tipula* spp.;
- from the order *Siphonaptera,* for example, *Ceratophyllus* spp. and *Xenopsylla cheopis;* and
- from the infraclass *Thysanura,* order *Zygentoma,* for example, *Lepisma saccharina.*
It is understood by the skilled person that certain genera and species are also known by their synonyms.

Preferably, said insect is considered a pest. A "pest", as used here, is an organism that is detrimental to humans or human concerns. "Pest insect" includes, but is not limited to agricultural pest insects, including but not limited to caterpillars, beetles, aphids, grasshoppers, stinkbugs, thrips, white flies, etc., household pest insects, such as flies, wasps, cockroaches, ants, house crickets, bed bugs, wood worms, mealworm beetles, earwigs, moths, silverfish, termites, etc., blood-feeding pest insects such as mosquitoes, fleas and lice, and disease vectors, such as malaria mosquitoes and tsetse flies. More preferably, said pest insect is an agricultural pest insect.

### [INSECT CONTROL]

To "control" or "controlling" insects means to inhibit or reduce the ability of one or more insects to feed, grow, develop, survive, and/or reproduce, or to limit insect-related damage. To "control" insects may mean any of the following non-limiting list: killing the insect, decreasing the insect's survival and/or longevity, decreasing the insect's fecundity and/or fertility, decreasing or arresting the insect's feeding, decreasing or arresting the insect's growth, decreasing or arresting the insect's development, deterring the insect, or preventing infestation by the insect. Preferably "controlling" insects means killing the insects. Thus in a preferred embodiment, the insect-controlling polypeptides of the invention are insecticidal polypeptides.

The "percentage insect control" means the percentage insect control in a population of insects that is treated or contacted with the insecticidal polypeptide as disclosed herein. As an example, in the case that insect control means insect killing, the "percentage insect control" means the percentage of insects killed. As another example, in the case that insect control means decreasing the insect's survival and/or longevity, the "percentage insect control" means the decrease in average survival or longevity in the population. As another example, in the case that insect control means decreasing the insect's fecundity and/or fertility, this means the percentage decrease in offspring of the population. It must be clear to the skilled artisan how the "percentage insect control" within a population of insects can be calculated. In a preferred embodiment, the percentage insect control is at least 50%. In a more preferred embodiment, the percentage insect control is at least 60%, or at least 70%. In an even more preferred embodiment, the percentage insect control is at least 75%, or at least 80%, or at least 85%, or at least 90 %, or at least 95%. In a most preferred embodiment, the percentage insect control is at least 97%, or at least 99%, or 100%.

### [BINDING DOMAINS]

A binding domain as disclosed herein is a domain comprised in a macromolecule and capable of binding a binding site. In one embodiment, the macromolecule is a polypeptide. The terms "specifically binding", "preferentially binding", "specific binding", and "preferential binding", as used herein, generally refers to the ability of a binding domain to preferentially bind to a particular binding site that is present in a homogeneous mixture of different molecules. In certain embodiments, a specific binding interaction will discriminate between desirable and undesirable binding sites in a sample, in some embodiments more than about 10 to 100-fold or more (e.g., more than about 10³-fold, or more than about 10⁴-fold, or more than about 10⁵-fold, or more than about 10⁶-fold).

The term "affinity", as used herein, refers to the degree to which a binding domain binds to a particular binding site so as to shift the equilibrium of binding domain and binding site toward the presence of a complex formed by their binding. Thus, for example, where an antigen and antibody (fragment) are combined in relatively equal concentration, an antibody (fragment) of high affinity will bind to the available antigen so as to shift the equilibrium toward high concentration of the resulting complex. The dissociation constant is commonly used to describe the affinity between the protein binding domain and the binding site. Typically, the dissociation constant is lower than 10⁻⁵ M. Preferably, the dissociation constant is lower than 10⁻⁶ M, more preferably, lower than 10⁻⁷ M. Even more preferably, the dissociation constant is lower than 10⁻⁸ M. Most preferably, the dissociation constant is lower than 10⁻⁹ M.

Thus, in certain embodiments, a binding domain as disclosed herein is said to be specific for a binding site of interest as opposed to a second site when it binds to the binding site of interest with an affinity that is at least 5 times, such as at least 10 times, such as at least 100 times, preferably at least 10³ times, more preferably at least 10⁴ times, or most preferably at least 10⁵ times higher than the affinity with which that binding domain as disclosed herein binds to the second site. Thus, in certain embodiments, a binding domain as disclosed herein is said to be specific for a binding site of interest as opposed to a second site when the dissociation constant for its binding to the binding site of interest is is at least 5 times, such as at least 10 times, such as at least 100 times, preferably at least 10³ times, more preferably at least 10⁴ times, or most preferably at least 10⁵ times lower than the dissociation constant for its binding to the second site. Accordingly, in certain embodiments, when a binding domain as disclosed herein is said to be "specific for" a binding site as opposed to a second site, it may specifically bind to (as defined herein) the binding site of interest, but not to the second site.

Preferably, the binding domain is capable of binding to said binding site under conditions that are reminiscent of conditions in the field or in a greenhouse or in a human inhabited environment. More preferably, said binding domain is maintaining its binding functionality in a biological control formulation and/or in an agrochemical formulation (both as defined hereinafter).

Binding domains are known to the person skilled in the art and include, but are not limited to antigen binding domains, such as those in heavy chain antibodies (hcAb), single domain antibodies (sdAb), minibodies (Tramontano et al., 1994), the variable domain of camelid heavy chain antibodies (VHH) (WO 1994/025591 A1 the variable domain of the new antigen receptors (VNAR) (Nuttall et al., 2003), engineered CH2 domains (nanoantibodies) (Dimitrov, 2009) and alphabodies (WO 2010/066740 A1), and functional fragments thereof.

Preferably, said binding domain is an antigen binding domain. An "antigen binding domain", as used herein, is a binding domain that binds to an antigen. Preferably, said antigen binding domain is comprised in an antigen binding domain comprising an amino acid sequence that comprises 4 framework regions and 3 complementary determining regions, according to Kabat. Binding domains comprising 4 FRs and 3 CDRs, preferably in a sequence FR1-CDR1-FR2-CDR2-FR3-CDR3-FR4, are known to the person skilled in the art and have been described, as a non-limiting example in Wesolowski et al. (Wesolowski et al., 2009). The length of the CDR3 loop is strongly variable and can vary from 0, preferably from 1, to more than 20 amino acid residues, preferably up to 25 amino acid residues.

Preferably, said antigen binding domains are derived from camelid antibodies, preferably from heavy chain camelid antibodies, devoid of light chains, such as variable domains of heavy chain camelid antibodies (VHH). Camelid antibodies, and the VHH derived sequences are known to the person skilled in the art. Camelid antibodies have been described, amongst others in WO 1994/004678 A1 and in WO 1994/025591 A1. Those antibodies are easy to produce, and are far more stable than classical antibodies, which provides a clear advantage for stable binding to the insect in a natural environment, where the binding conditions cannot be controlled.

Most preferably, said VHH comprises, preferably consists of, a specified sequence of a VHH, or homologues thereof. Homologues, as used here, are sequences wherein each or any framework region and each or any complementary determining region shows at least 80% homology, preferably at least 85% homology, more preferably 90% homology, even more preferably 95% homology with the corresponding region in the reference sequence (i.e. FR1 homologue versus FR1 reference, CDR1 homologue versus CDR1 reference, FR2 homologue versus FR2 reference, CDR2 homologue versus CDR2 reference, FR3 homologue versus FR3 reference, CDR3 homologue versus CDR3 reference and FR4 homologue versus FR4 reference; FR and CDR definitions according to Kabat) as defined herein.

The binding domains of the invention include functional fragments of the above-described binding domains. It is clear to the skilled person that "functional fragment" means any fragment that has the same or similar functionality of the binding domain, being binding to the binding site and/or insect control. In the preferred embodiment where the binding domain is a VHH, a functional fragment thereof will usually contain at least some of the amino acid residues that form at least one of the complementary determining regions.

### [BINDING SITES]

A "binding site" of the target insect as disclosed herein is a molecular structure or part of a molecular structure of the target insect to which the binding domain comprised in the insect-controlling polypeptide binds. In a preferred embodiment, the binding site is located inside the insect. As used herein, inside the insect includes all molecules, structures, tissues and organs of an insect which are not at the surface of an intact insect. The "surface", as used herein, can be any surface as it occurs on the outside of an insect; however it excludes histological preparations of insects. In a more preferred embodiment, the binding site is located in the digestive tract of the target insect. Digestive tract, alimentary canal and gut as used herein are used interchangeably. The digestive tract of an insect includes the foregut or stomadeum (including any of buccal cavity, salivary glands, pharynx, esophagus, crop, and proventriculus), the midgut or mesenteron (including any of gastric caeca and ventriculus), and the hindgut or proctodeum (including any of Malpighian tubules, pylorus, ileum, rectum, and anus). In an even more preferred embodiment, the binding site is located in the midgut and/or hindgut of the target insect, more preferably the midgut of the target insect. Preferably, the binding site is specific for the midgut, which means the binding site is predominantly or only present in the midgut. The binding site can be comprised on or in the epithelium and be located on or in columnar cells, on or in goblet cells, or be part of the peritrophic membrane, if present. In a more preferred embodiment, the binding site is present on the columnar cells of the epithelium. In a more preferred embodiment, the binding site is located at the brush border of microvilli of the columnar cells. In an even more preferred embodiment, the binding site is cell membrane-associated. As used herein, "membrane-associated" means that it is directly of indirectly in contact with the cell membrane of insect cells. Examples of cell membrane-associated binding sites are cell membrane proteins, which include integral membrane proteins that are permanently anchored to or part of the membrane, and peripheral membrane proteins that are only temporarily attached to the lipid bilayer or to other integral proteins. The integral membrane proteins are classified as transmembrane proteins that span across the membrane with one or more transmembrane domains, and integral monotopic proteins that are attached to only one side of the membrane. Membrane receptor proteins relay signals between the cell's internal and external environments. Transport proteins move molecules and ions across the membrane. Membrane enzymes may have many activities, such as oxidoreductase, transferase or hydrolase. Cell adhesion proteins are proteins located on the cell surface involved in binding with other cells or with the extracellular matrix (ECM) in the processes of cell adhesion and cell recognition. Other examples of binding sites directly associated with the cell membrane are lipids, glycolipids, and other lipophilic molecules such as sterols which are integrated in the lipid layer. Binding sites indirectly associated with the cell membrane include molecules which are not directly associated with the cell membrane, but which are bound non-covalently to molecules directly associated with the cell membrane.

In a preferred embodiment, the binding site is selected from the group consisting of Cation-Amino Acid Transporter/Channel 1 (CAATCH1), K⁺-coupled amino acid transporter (KAAT1), V-ATPase subunit a, V-ATPase subunit c, V-ATPase subunit e, Insect Intestinal Mucins 14 and 22 (IIM14 and IIM22), ATP-binding cassette transporter subfamily H member 1 (ABCH1), (Snakeskin) SSK, Snakeskin-associated MESH, goblet cell apical membrane K⁺/2H⁺ antiporter, goblet cell apical membrane Cl⁻ channel, and columnar cell apical membrane Cl⁻/HCO₃⁻ antiporter. Putative orthologues in *Drosophila melanogaster* and in some Lepidopteran species are given in Table 1.

**Table 1: Preferred binding site orthologs. The prototype amino acid sequence used for searching ortholoques for each preferred binding site molecule is underlined.**

| **Binding site molecule** | ***Lepidoptera* putative orthologues GenBank or RefSeq entries (E-value)** | ***D. melanogaster* putative orthologues CG numbers (E-value)** |
|---|---|---|
| CAATCH1 | AAF18560.1 | CG15279 (1.30972e-164) |
| | AAC24190.1 (0.0) | CG3252 NAAT1 (1.84394e-164) |
| | XP_021190151.1 (0.0) | |
| | XP_014367537.1 (0.0) | |
| | XP_014367544.1 (0.0) | |
| | XP_004927040.1 (0.0) | |
| | NP_001124343.1 (0.0) | |
| | XP_013199759.1 (0.0) | |
| | XP_013199760.1 (0.0) | |
| | XP_011562370.1 (0.0) | |
| | XP_011562371.1 (0.0) | |
| | JAT79914.1 (0.0) | |
| | EHJ64672.1 (0.0) | |
| KAAT1 | AAC24190.1 | CG15279 (1.30972e-164) |
| | AAF18560.1 (0.0) | CG3252 NAAT1 (1.84394e-164) |
| | XP_021190151.1 (0.0) | |
| | XP_014367537.1 (0.0) | |
| | XP_014367544.1 (0.0) | |
| | XP_004927040.1 (0.0) | |
| | NP_001124343.1 (0.0) | |
| | XP_013199759.1 (0.0) | |
| | XP_013199760.1 (0.0) | |
| | XP_011562370.1 (0.0) | |
| | XP_011562371.1 (0.0) | |
| | JAT79914.1 (0.0) | |
| | EHJ64672.1 (0.0) | |
| V-ATPase subunit a | CAA45537.1 | CG12403 Vha68-1 (0.0) |
| | NP_001091829.1 (0.0) | CG3762 Vha68-2 (0.0) |
| | EHJ78293.1 (0.0) | |
| | XP_013172918.1 (0.0) | |
| | ADP23923.1 (0.0) | |
| | XP_013194364.1 (0.0) | |
| | AJQ81220.1 (0.0) | |
| | AKK21114.1 (0.0) | |
| | XP_011562234.1 (0.0) | |
| | XP_011562236.1 (0.0) | |
| V-ATPase subunit c | CAB55498.1 | CG8048 Vha44 (0.0) |
| | NP_001298659.1 (0.0) | |
| | AIJ50380.1 (0.0) | |
| | NP_001040138.1 (0.0) | |
| | XP_013193253.1 (0.0) | |
| | XP_011553182.1 (0.0) | |
| | XP_011553183.1 (0.0) | |
| | EHJ73191.1 (0.0) | |
| V-ATPase subunit e | CAA47610.1 | CG1088 Vha26 (2.84585e-94) |
| | NP_001040451.1 (7e-155) | |
| | EHJ78213.1 (1e-150) | |
| | XP_013186293.1 (3e-150) | |
| | NP_001299572.1 (2e-148) | |
| | KOB69744,1 (2e-143) | |
| | NP_001292461.1 (2e-140) | |
| IIM14/IIM22 | AAC47556.1 | none |
| | AAC47557.1 | |
| | AAL17912.1 (7e-90) | |
| ABCH1 | AKC96438.1 | CG9990 (0.0) |
| | XP_011547735.1 (0.0) | CG33970 (0.0) |
| | XP_011547736.1 (0.0) | |
| | XP_013165572.1 (0.0) | |
| | XP_013165571.1 (0.0) | |
| | XP_013197222.1 (0.0) | |
| | XP_013197216.1 (0.0) | |
| | XP_004933088.1 (0.0) | |
| | XP_004933046.1 (0.0) | |
| | EHJ78794.1 (0.0) | |
| SSK | ANX99820.1 | CG6981 SSK (7.27639e-47) |
| | XP_013199749.1 (7e-69) | |
| | XP_013143312.1 (2e-68) | |
| | H9JBU5.2 (4e-68) | |
| | XP_004932562.1 (2e-65) | |
| | EHJ68391.1 (1e-64) | |
| | XP_011561759.1 (5e-63) | |
| | KOB74629,1 (6e-50) | |
| MESH | ANX99821.1 | CG31004 MESH (0.0) |
| | JAT87391.1 (0.0) | |
| | JAT90784.1 (0.0) | |
| | XP_013191386.1 (0.0) | |
| | XP_013191387.1 (0.0) | |
| | XP_013149247.1 (0.0) | |
| | XP_013149255.1 (0.0) | |
| | XP_004925419.1 (0.0) | |
| | H9JIQ1.1 (0.0) | |
| | XP_004925418.1 (0.0) | |
| | EHJ74925.1 (0.0) | |
| | XP_011551827.1 (0.0) | |
| | KOB67705.1 (0.0) | |

### [POLYPEPTIDES COMPRISING AT LEAST ONE BINDING DOMAIN]

In certain embodiments, the insect-controlling polypeptides of the invention comprise one binding domain. In other embodiments, the insect-controlling polypeptides comprise one or more additional binding domains. Thus the insect-controlling polypeptides comprise one, two, three, four, five or more binding domains. The additional binding domains may be identical to or different from the first binding domain and from each other. The locations of the binding sites bound by the additional binding domains may be identical to or different from the location of the binding site bound by the first binding domain. The binding sites bound by the additional binding domains may be identical to or different from the binding site bound by the first binding domain.

### [TOXIN MOIETIES]

In certain embodiments, the insect-controlling polypeptides of the invention control the insect by virtue of the one or more binding domains present in the polypeptides. Thus binding of the binding domain(s) to the binding site(s) is sufficient for the insect-controlling action of the polypeptides. In other embodiments, in addition to the one or more binding domains that bind the binding site(s), the insect-controlling polypeptides further comprise one or more toxin moieties which are different from the binding domains of the present invention. As used herein, "toxin moiety" and "toxin" are used interchangeably and mean any molecule which has insect-controlling capability. As such, "toxin moiety" can be any insect-controlling polypeptide, polynucleotide, small molecule, etc.

Examples for useful toxin moieties include δ-endotoxins such as Cry1A, Cry1B, Cry1C, Cry1D, Cry1E, Cry1F, Cry1G, Cry1H, Cry1I, Cry1J, Cry1K, Cry2A, Cry7B, Cry8D, Cry9A, Cry9B, Cry9C, Cry9D, Cry9E, Cry15A, Cry22A, Cry32A, Cry51A, Cyt1A (Crickmore et al., 1998; van Frankenhuyzen, 2009). Other useful pore-forming toxin (PFT) moieties are toxins including but not limited to colicins (such as colicin E1, colicin la, colicin A, colicin N), actinoporins (such as equinatoxin II, sticholysin II, fragaceatoxin C), ClyA family toxins (such as cytolysin A, non-haemolytic tripartite enterotoxin, haemolysin BL), haemolysins (such as α-haemolysin, γ-haemolysin, leukocidins, nectrotic enteritis toxin B, δ-toxin, *Vibrio cholerae* cytolysin, *Vibrio vulnificus* haemolysin), aerolysin family toxins (such as aerolysin, α-toxin, hydralysin, ε-toxin, enterotoxin, haemolytic lectin, kysenin), cholesterol-dependent cytolysins (such as perfringolysin, suilysin, intermedilysin, listeriolysin O, lectinolysin, anthrolysin, streptolysin), membrane attack complex components/perforins (such as Plu-MACPF, Bth-MACPF), and repeats-in-toxins (such as HlyA, bifunctional haemolysin-adenylyl cyclase toxin, MARTX) (Dal Peraro and van der Goot, 2016).

Other useful toxins include spider toxins and scorpion toxins.

Insect-controlling double-stranded RNAs are also included in "toxin moiety" in that they can control insects by interfering with the expression of one or more genes in the insect, inhibition or down-regulation of which gene(s) results in inhibition or reduction of the ability of the insect to feed, grow, develop, survive, and/or reproduce, or to limit insect-related damage.

### [FORMULATIONS]

It is understood that the compositions for agrochemical, pharmaceutical, veterinary, household, or other insect controlling use as disclosed herein are stable, both during storage and during utilization, meaning that the integrity of the composition is maintained under storage and/or utilization conditions of the composition, which may include elevated temperatures, freeze-thaw cycles, changes in pH or in ionic strength, UV-irradiation, presence of harmful chemicals and the like. More preferably, the at least one polypeptide as disclosed herein remains stable in the composition, meaning that the integrity and the insect-controlling activity of the polypeptide is maintained under storage and/or utilization conditions of the composition, which may include elevated temperatures, freeze-thaw cycles, changes in pH or in ionic strength, UV-irradiation, presence of harmful chemicals and the like. Most preferably, said at least one polypeptide remains stable in the composition when the composition is stored at ambient temperature for a period of two years or when the composition is stored at 54°C for a period of two weeks. Preferably, the compositions as disclosed herein retain at least about 70% activity, more preferably at least about 70% to 80% activity, most preferably about 80% to 90% activity or more. Optionally, the at least one polypeptide may be comprised in a carrier, as defined, to protect the polypeptide from harmful effects caused by other components in the composition or from harmful effects during storage or during application. Examples of suitable carriers include, but are not limited to alginates, gums, starch, β-cyclodextrins, celluloses, polyurea, polyurethane, polyester, microbial cells or clay.

The insect-controlling polypeptide as disclosed herein may be the only active substance in the composition according to the invention; however, it is also possible that the composition comprises one or more additional insect-controlling agents in addition to the polypeptide (or the at least one, at least two or at least three polypeptides or as disclosed herein). Such additional insect-controlling agents may have a different effect on insects as the polypeptide, they may have a synergistic effect with the polypeptide, or they may even modify the activity of the polypeptide.

In preferred embodiments, the additional insect-controlling agents are biochemical insect-controlling agents which are toxic to the same insects, such as a patatin, a *Bacillus thuringiensis* insecticidal protein, a *Xenorhabdus* insecticidal protein, a *Photorhabdus* insecticidal protein, a *Bacillus laterosporous* insecticidal protein, a *Bacillus bombysepticus* insecticidal protein, a *Bacillus sphaericus* insecticidal protein, insect-controlling double-stranded RNAs, or insect-controlling small molecules. The compositions may be formulated prior to administration in an appropriate means such as lyophilized, freeze-dried, desiccated, or in an aqueous carrier, medium or suitable diluent, such as saline or other buffer. The formulated compositions may be in the form of a dust or granular material, or a suspension in oil (vegetable or mineral), or water or oil/water emulsions, or as a wettable powder, or in combination another other carrier material suitable for application.

The compositions as disclosed herein may be applied to the environment of the target pest, for example onto the foliage of the plant or crop to be protected or treated, on the skin or hair of the animal to be protected or treated, or on the object to be protected, by conventional methods, preferably by spraying. The pesticidal compositions as disclosed herein may comprise a pest attractant. The pesticidal compositions may be formulated for either systemic or topical use. The strength and duration of application may be set with regard to conditions specific to the particular pest(s), crop(s), animals and objects to be protected or treated, and particular environmental conditions. Other application techniques, e.g., dusting, sprinkling, soaking, soil injection, seed coating, seedling coating, object coating, spraying, aerating, misting, atomizing, and the like, are also feasible and may be required under certain circumstances. These application procedures are also well-known to those of skill in the art.

### [FORMULATIONS FOR AGRONOMICAL APPLICATION]

Agrochemical compositions may occur in any type of formulation, preferred formulations are powders, wettable powders, wettable granules, water dispersible granules, emulsions, emulsifiable concentrates, dusts, suspensions, suspension concentrates, suspoemulsions (mixtures of suspensions and emulsions), capsule suspensions, aqueous dispersions, oil dispersions, aerosols, pastes, foams, slurries or flowable concentrates.

Suitable additional agrochemicals can be herbicides, insecticides, fungicides, nematicides, acaricides, bactericides, viricides, plant growth regulators, safeners and the like and include, but are not limited to glyphosate, paraquat, metolachlor, acetochlor, mesotrione, 2,4-D, atrazine, glufosinate, sulfosate, fenoxaprop, pendimethalin, picloram, trifluralin, bromoxynil, clodinafop, fluroxypyr, nicosulfuron, bensulfuron, imazetapyr, dicamba, imidacloprid, thiamethoxam, fipronil, chlorpyrifos, deltamethrin, lambda-cyhalothrin, endosulfan, methamidophos, carbofuran, clothianidin, cypermethrin, abamectin, diflufenican, spinosad, indoxacarb, bifenthrin, tefluthrin, azoxystrobin, thiamethoxam, tebuconazole, mancozeb, cyazofamid, fluazinam, pyraclostrobin, epoxiconazole, chlorothalonil, copper fungicides, trifloxystrobin, prothioconazole, difenoconazole, carbendazim, propiconazole, thiophanate, sulphur, boscalid and other known agrochemicals or any suitable combination(s) thereof.

Agrochemical compositions comprising the polypeptides, polynucleotides, cells, vectors, etc., as disclosed herein can be formulated with an agriculturally acceptable carrier. Suitable agricultural carriers can be solid or liquid and are well known in the art. The term "agriculturally acceptable carrier" covers all adjuvants, e.g., inert components, dispersants, surfactants, tackifiers, binders, etc. that are ordinarily used in insecticide formulation technology; these are well known to those skilled in insecticide formulation. The formulations may be mixed with one or more solid or liquid adjuvants and prepared by various means, e.g., by homogeneously mixing, blending and/or grinding the insecticidal composition with suitable adjuvants using conventional formulation techniques.

### [FORMULATIONS FOR MEDICAL OR VETERINARY APPLICATION]

In some embodiments the compositions of the present invention are formulated for prophylactic or therapeutic treatment of human or animal subjects. Therefore, the present invention relates to formulations, wherein said formulations are suitable for topical, enteral or parenteral administration to a subject, e.g., a human subject or animal subject. The formulation can be administered to the subject topically (e.g. in the form of a spray, an ointment, a shampoo, a powder, a lotion, a cream, a gel, an impregnated patch), orally, by injection (e.g., intravenous, subcutaneous, intramuscular or intraperitoneal) or by inhalation.

### [FORMULATIONS FOR OBJECT APPLICATION AND BAITS]

In some embodiments the compositions of the present invention are formulated for preventive or curative treatment of non-living objects. Such formulations can be in the form of e.g. a spray, a powder, a cream, a liquid, a paste, a coating, a pellet, a gel, etc. The compositions of the present invention may be applied to the object to protect or treat, after which the insect by contacting the object will contact the composition.

In one embodiment, the composition is in the form of a bait. The bait is designed to lure the insect to come into contact with the composition. Upon coming into contact therewith, the composition is then taken up by the insect, by ingestion for example. Said bait may comprise a food substance, such as a protein based food, for example fish meal. Boric acid may also be used as a bait. The bait may depend on the species being targeted. An attractant may also be used. The attractant may be a pheromone, such as a male or female pheromone for example. As an example, the pheromones referred to in the book "Insect Pheromones and their use in Pest Management" (Howse et al, Chapman and Hall, 1998) may be used in the invention. The attractant acts to lure the insect to the bait, and may be targeted for a particular insect or may attract a whole range of insects. The bait may be in any suitable form, such as a solid, paste, pellet or powdered form.

The bait may also be carried away by the insect back to the colony. The bait may then act as a food source for other members of the colony, thus providing an effective control of a large number of insects and potentially an entire insect pest colony.

Additionally, compositions which come into contact with the insect may remain on the cuticle of the insect. When cleaning, either an individual insect cleaning itself or insects cleaning one another, the compositions may be ingested and can thus mediate their effects in the insect. This requires that the composition is sufficiently stable such that the insect-controlling polypeptide remains intact and capable of controlling the insect even when exposed to external environmental conditions for a length of time, which may be a period of days for example.

The baits may be provided in a suitable "housing" or "trap". Such housings and traps are commercially available and existing traps may be adapted to include the compositions of the invention. Any housing or trap which may attract an insect to enter it is included within the scope of the invention. The housing or trap may be box-shaped for example, and may be provided in preformed condition or may be formed of foldable cardboard for example. Suitable materials for a housing or trap include plastics and cardboard, particularly corrugated cardboard. Suitable dimensions for such a housing or trap are, for example, 7-15 cm wide, 15-20 cm long and 1 -5 cm high. The inside surfaces of the traps may be lined with a sticky substance in order to restrict movement of the insect once inside the trap. The housing or trap may contain a suitable trough inside which can hold the bait in place. A trap is distinguished from a housing because the insect can not readily leave a trap following entry, whereas a housing acts as a "feeding station" which provides the insect with a preferred environment in which they can feed and feel safe from predators.

### [METHODS FOR CONTROLLING INSECTS]

Provided herewith are methods for controlling insects. In one embodiment, the methods comprises the step of contacting or feeding the insect with at least one polypeptide, nucleic acid, DNA construct, host cell, or composition according to the present invention. After uptake or ingestion of the at least one polypeptide, nucleic acid, DNA construct, host cell, or composition by the insect, the binding domain of the insect-controlling polypeptide binds to the binding site of the insect, after which it can exert its insect-controlling activity.

Methods for controlling insects include methods for prevention or treatment of plants and plant parts, methods of medical or veterinary protection or treatment, and methods of object protection or treatment. Depending on the application, contacting or feeding the insect with at least one polypeptide, nucleic acid, DNA construct, host cell, or composition according to the present invention.

### [METHODS FOR PREVENTION OR TREATMENT OF INSECT INFESTATIONS OF PLANTS AND PLANT PARTS]

Also provided are methods for preventing or treating infestation by an insect of a plant or plant part. In one embodiment, the method comprises the step of applying to the plant or plant part at least one polypeptide, nucleic acid, DNA construct, host cell, host cell derivatives, or composition according to the present invention. Thus by coming in contact with the plant or plant part, or feeding on the plant or plant part, the insect-controlling polypeptide is taken up or ingested by the insect. In another embodiment, a bait comprising at least one polypeptide, nucleic acid, DNA construct, host cell, or composition according to the present invention is placed in, near, or in between, the plants or plant part to lure the insects, after which the bait is taken up or ingested by the insect.

"Plant" as used herein, means live plants (Kingdom Plantae) and live plant parts, including fresh fruit, vegetables and seeds. Also, the term "plant" as used herein encompasses whole plants, ancestors and progeny of the plants, and plant parts, including seeds, fruits, shoots, stems, bark, leaves, roots (including tubers), flowers, buds, and tissues and organs. The term "plant" also encompasses plant cells, suspension cultures, callus tissue, embryos, meristematic regions, gametophytes, sporophytes, pollen and microspores.

The term "plant tissue" as used herein refers to a group of similar plant cells from the same origin that together carry out a specific function. Examples of plant tissues include meristematic tissue, protective tissue, parenchyma, sclerenchyma, collenchyma, xylem, and phloem.

The plants to be protected from or treated for insect infestation may be crops. "Crop" as used herein means a plant species or variety that is grown to be harvested as food, livestock fodder, fuel raw material, or for any other economic purpose. As a non-limiting example, said crops can be maize, cereals, such as wheat, rye, barley and oats, sorghum, rice, sugar beet and fodder beet, fruit, such as pome fruit (e.g. apples and pears), citrus fruit (e.g. oranges, lemons, limes, grapefruit, or mandarins), stone fruit (e.g. peaches, nectarines or plums), nuts (e.g. almonds or walnuts), soft fruit (e.g. cherries, strawberries, blackberries or raspberries), the plantain family, grapevines, leguminous crops, such as beans, lentils, peas and soya, oil crops, such as sunflower, safflower, rapeseed, canola, castor or olives, cucurbits, such as zucchini, cucumbers, melons or pumpkins, fiber plants, such as cotton, flax or hemp, fuel crops, such as sugarcane, miscanthus, yatropha or switchgrass, vegetables, such as potatoes, tomatoes, peppers, lettuce, spinach, onions, carrots, egg-plants, asparagus or cabbage, ornamentals, such as flowers (e.g. petunias, pelargoniums, roses, tulips, lilies, or chrysanthemums), shrubs (e.g. boxwood), broad-leaved trees (e.g. poplars or willows) and evergreens (e.g. conifers), grasses, such as lawn, turf or forage grass or other useful plants, such as coffee, tea, cocoa, tobacco, hops, pepper, rubber or latex plants.

In still a further aspect, the present invention provides treatment methods for post-harvest protecting or treating a harvested plant or a harvested plant part from an insect, at least comprising the step of applying before or after harvesting to the plant or plant part, at least one polypeptide, nucleic acid, DNA construct, host cell, or composition according to the present invention.

### [METHODS OF MEDICAL OR VETERINARY PROTECTION OR TREATMENT]

Also provided are (prophylactic/preventive) methods of protection of human and animal subjects from insects and (therapeutic/curative) methods of treatment of insect-infested human and animal subjects. The methods comprise the step of applying or administering to the human or animal subject a composition comprising the insect-controlling polypeptide of the present invention.

Also provided is the prophylactic/preventive and therapeutic/curative medical use of the insect-controlling polypeptide of the present invention.

Also provided is a polypeptide comprising a binding domain capable of binding a binding site of an insect for use in medicine.

In another embodiment, a bait comprising at least one polypeptide, nucleic acid, DNA construct, host cell, or composition according to the present invention is placed in the environment of the human or animal subject to lure the insects, after which the bait is taken up or ingested by the insect.

### [METHODS OF OBJECT PROTECTION OR TREATMENT]

Also provided are methods of protection of objects from insects or methods of treatment of insect-infected objects. The methods comprise the step of applying to the object a composition comprising the insect-controlling polypeptide of the present invention. Thus by coming in contact with the object, or feeding on the object, the insect-controlling polypeptide is taken up or ingested by the insect. Alternatively, a bait comprising a composition comprising the insect-controlling polypeptide of the present invention is placed near the object to be protected to lure the insects, after which the bait is taken up or ingested by the insect.

### [CONSTRUCTS, VECTORS, HOST CELLS, TRANSGENIC PLANTS]

Further provided are nucleic acids comprising a polynucleotide encoding an insect-controlling polypeptide of the invention. These nucleic acids can be comprised in a nucleic acid construct. As used herein, the terms "genetic construct" and "nucleic acid construct" are used interchangeably. The nucleic acids as disclosed herein may be synthetic or semi-synthetic sequences, nucleic acids that have been isolated from a library (and in particular, an expression library), nucleic acids that have been prepared by PCR using overlapping primers, or nucleic acids that have been prepared using techniques for DNA synthesis known per se. In certain embodiments, the polynucleotide or nucleic acid construct may comprise have nucleic acid sequence as specified herein.

The nucleic acid constructs as disclosed herein may be DNA or RNA, and are preferably double-stranded DNA. The nucleic acid constructs of the invention may also be in a form suitable for transformation of the intended host cell or host organism in a form suitable for integration into the genomic DNA of the intended host cell or in a form suitable for independent replication, maintenance and/or inheritance in the intended host organism. For instance, the nucleic acid constructs of the invention may be in the form of a vector, such as for example a plasmid, phagemid, cosmid, BAC, TAC, YAC, a viral vector or transposon. In particular, the nucleic acid construct may be an expression construct, i.e., a nucleic acid construct that can provide for expression *in vitro* and/or *in vivo* (e.g. in a suitable host cell, host organism and/or expression system).

Accordingly, in another further aspect, the present invention also provides nucleic acid constructs comprising a nucleic acid comprising a polynucleotide encoding an insect-controlling polypeptide of the invention.

Also disclosed are nucleic acid constructs in which the nucleic acid comprising the polynucleotide encoding an insect-controlling polypeptide of the invention is operably linked to at least one regulatory polynucleotide capable of driving expression of the nucleic acid. In some embodiments, the nucleic acid construct comprises the following operably linked regulatory polynucleotides: a) a promoter expressible in a host cell or expression system, b) a nucleic acid which when transcribed yields a mRNA molecule capable of being translated into a polypeptide and, c) a region comprising a transcription terminator and/or polyadenylation signals functioning in said host cell or expression system. The term "operably linked" as used herein refers to a functional linkage between the promoter sequence and the gene of interest, such that the promoter sequence is able to initiate transcription of the gene of interest. In some embodiments, the regulatory polynucleotides are heterologous, meaning that they are not normally operatively linked to the associated nucleic acid sequence as found in nature.

In certain embodiments, the nucleic acid constructs may comprise a promoter suitable for expression in plants, plant tissue or plant cells. In other embodiments, the nucleic acid constructs may comprise a promoter suitable for expression in bacterial cells. In still other embodiments, the nucleic acid constructs may comprise a promoter suitable for expression in fungal cells. In still other embodiments, the nucleic acid constructs may comprise a promoter suitable for expression in animal cells. In some embodiments, the nucleic acid constructs may comprise an inducible promoter.

The term "plant promoter" or "promoter suitable for expression in plants" are used interchangeably herein and refer to a regulatory polynucleotide which mediates the expression of a operably linked nucleic acid in plant cells. Plant promoters comprise polynucleotide s which are able to direct the expression of a nucleic acid in a plant. Examples of plant promoters are constitutive promoters which are transcriptionally active during most, but not necessarily all, phases of growth and development and under most environmental conditions, in at least one cell, tissue or organ, other promoters are inducible promoters, other examples are plant tissue-specific and plant cell-specific promoters. The terms "plant tissue-specific" and "plant cell specific promoter" refer to promoters which are transcriptionally active in a specific type of plant tissues or plant cells. The term "inducible promoter" refers to promoters which allow regulating gene expression levels at particular stages of plant development and in particular tissues of interest. Examples of inducible systems include AlcR/AlcA (ethanol inducible); GR fusions, GVG, and pOp/LhGR (dexamethasone inducible); XVE/OlexA (beta-estradiol inducible); and heat shock induction.

The term "transcription terminator" encompasses a regulatory polynucleotide which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or from any other eukaryotic gene.

The terms "transgenic plant" and "transgenic plant cell" generally refers to plants and plant cells that have been genetically engineered to create plants and plant cells with new characteristics. A transgenic plant or plant cell can also be identified as a genetically modified organism (GMO).

The term "transgenic plant" also encompasses commodity products derived from the transgenic plant or plant cell, wherein the commodity product comprises a detectable amount of the insect-controlling polypeptide as disclosed herein, and wherein the commodity product is selected from the group consisting of plant biomass, oil, meal, food, animal feed, flour, flakes, bran, lint, fiber, paper, protein, starch, silage, hulls, and processed seed, and wherein optionally the commodity product is non-regenerable.

In certain embodiments, the plant may be selected from the group consisting of maize, soybean, alfalfa, cotton, sunflower, Brassica oil seeds such as *Brassica napus* (e.g. canola, rape- seed), *Brassica rapa, Brassica juncea* (e.g. (field) mustard) and *Brassica carinata, Arecaceae* sp. (e.g. oilpalm, coconut), rice, wheat, sugar beet, sugar cane, oats, rye, barley, millet and sorghum, triticale, flax, nuts, grapes and vine and various fruit and vegetables from various botanic taxa, e.g. *Rosaceae* sp. (e.g. pome fruits such as apples and pears, but also stone fruits such as apricots, cherries, almonds, plums and peaches, and berry fruits such as strawberries, raspberries, red and black currant and gooseberry), *Ribesioidae* sp., *Juglandaceae* sp., *Betulaceae* sp., *Anacardiaceae* sp., *Fagaceae* sp., *Moraceae* sp., Oleaceae sp. (e.g. olive tree), *Actinidaceae* sp., *Lauraceae* sp. (e.g. avocado, cinnamon, camphor), *Musaceae* sp. (e.g. banana trees and plantations), *Rubiaceae* sp. (e.g. coffee), *Theaceae* sp. (e.g. tea), *Sterculiceae* sp., *Rutaceae* sp. (e.g. lemons, oranges, mandarins and grapefruit); Solanaceae sp. (e.g. tomatoes, potatoes, peppers, capsicum, aubergines, tobacco), *Liliaceae* sp., *Compositae* sp. (e.g. lettuce, artichokes and chicory - including root chicory, endive or common chicory), *Umbelliferae* sp. (e.g. carrots, parsley, celery and celeriac), *Cucurbitaceae* sp. (e.g. cucumbers - including gherkins, pumpkins, watermelons, calabashes and melons), *Alliaceae* sp. (e.g. leeks and onions), *Cruciferae* sp. (e.g. white cabbage, red cabbage, broccoli, cauliflower, Brussels sprouts, pak choi, kohlrabi, radishes, horseradish, cress and Chinese cabbage), *Leguminosae* sp. (e.g. peanuts, peas, lentils and beans - e.g. common beans and broad beans), *Chenopodiaceae* sp. (e.g. Swiss chard, fodder beet, spinach, beetroot), *Linaceae* sp. (e.g. hemp), *Cannabeacea* sp. (e.g. cannabis), *Malvaceae* sp. (e.g. okra, cocoa), *Papaveraceae* (e.g. poppy), *Asparagaceae* (e.g. asparagus); useful plants and ornamental plants in the garden and woods including turf, lawn, grass and *Stevia rebaudiana,* and genetically modified types of these plants.

In certain embodiments, the plant may be a crop selected from the group consisting of field crops, grasses, fruits, vegetables, lawns, trees, and ornamental plants.

In certain embodiments, the plant may be a harvestable part of the plant selected from the group consisting of a fruit, a flower, a nut, a vegetable, a fruit or vegetable with inedible peel, preferably selected from avocados, bananas, plantains, lemons, grapefruits, melons, oranges, pineapples, kiwi fruits, guavas, mandarins, mangoes and pumpkin, is preferred, more preferably bananas, oranges, lemons and peaches, in particular bananas. In certain embodiments, the plant may be a cut flower of ornamental plants, preferably selected from *Alstroemeria,* carnation, Chrysanthemum, Freesia, *Gerbera, Gladiolus,* baby's breath (*Gypsophila* spp.), *Helianthus, Hydrangea, Lilium, Lisianthus,* roses and summer flowers. In certain embodiments, the plant may be cut grass or wood. In certain embodiments, the plant may be a plant used for research purposes such as *Arabidopsis,* corn, tobacco, or poplar.

Methods for the generation of transgenic plant including recombinant DNA techniques are well-known in the art.

In certain embodiments, the nucleic acid comprising the polynucleotide encoding the insect-controlling polypeptide as disclosed herein may comprise at least one sequence encoding a targeting signal for secretion, for location to the cytoplasm, or for location to cellular compartments or organelles, such as the ER lumen, the apoplast, the vacuole, or intra- and/or exterior membranes.

Examples of a targeting signal for secretion include the 2S2 signal peptide. Examples of a targeting signal for location to cellular compartments or organelles, such as the ER lumen include the ER retention signal (KDEL). In certain embodiments, the polynucleotide may comprise an ATG start codon for location to the cytoplasm.

The above disclosure is further described by means of the following non-limiting Examples and Figures, in which the figures show:

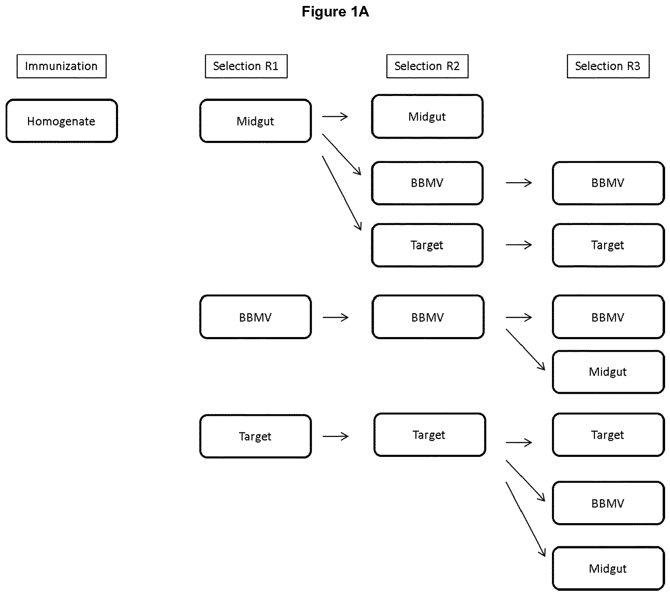

- **Figure 1A:**: Followed selection scheme based on the immunization with midgut homogenate.

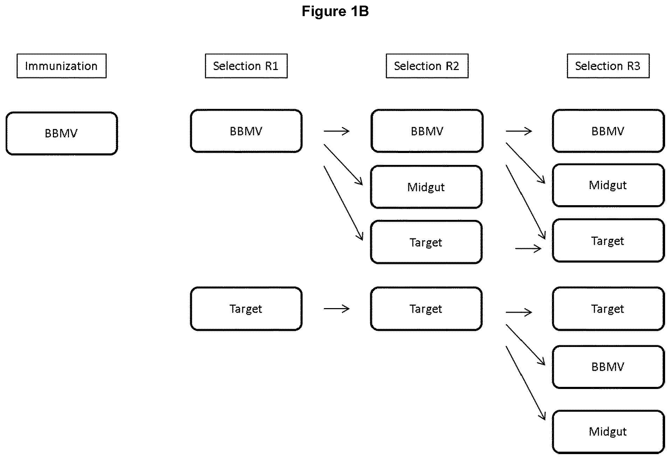

- **Figure 1B:**: Followed selection scheme based on the immunization with BBMVs.

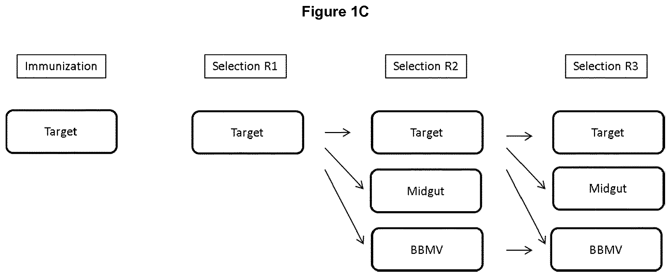

- **Figure 1C:**: Followed selection scheme based on the immunization with specific target.

### Examples

### Example 1: Preparation of antigen for immunization, selection and screening campaigns

***Preparation of insect homogenates -*** Colorado potato beetles (*Leptinotarsa decemlineata*) were dissected, exoskeletons and wings collected, and remainders discarded. Exoskeletons and wings were separately frozen in liquid nitrogen, ground with mortar and pestle, and fine powders collected. Colorado potato beetle larvae, Pea aphids (*Acyrthosiphon pisum*), and Tobacco Budworm larvae (*Heliothis virescens*) were frozen in liquid nitrogen, ground with mortar and pestle, and fine powders collected. Collected insect materials were resuspended in PBS and total protein concentrations of suspensions were determined with Bradford protein assay.

***Preparation of insect midgut homogenates and brush border membrane vesicles -*** L5 larvae from cotton bollworm (*Helicoverpa armigera*) and beet armyworm (*Spodoptera exigua*) are dissected. The midguts are isolated and peritrophic membranes are removed. Homogenates and brush border membrane vesicles (BBMVs) are prepared following the standard protocol for Lepidoptera of Wolfersberger et al. (Wolfersberger et al., 1987). Quality control is done by polyacrylamide gel electrophoresis (SDS-PAGE), enzymatic alkaline phosphatase assay and electron microscopy.

***Presenting specific molecular targets -*** A selection of proteins or biomolecules specific for Lepidoptera known to be displayed at the luminal side of the midgut, and expressed during the larvae stage are presented on cells or particles for immunization, selection and/or screening campaigns. Cell lines overexpressing or displaying selected molecular targets are derived from transfection of *Drosophila* S2, *Drosophila* Cl8, *Tribolium* TCA cells, Hi5, Bm5 or SF9 or the *Trichoplusia ni* midgut cell line Tn-MG1 (purchased from ATCC). Also proteoliposomes (Synthelis, La Tronche, France) and/or virus-like particles (VLPs, Integral Molecular, Philadelphia, USA) displaying the selected molecular targets are generated.

### Example 2: Generation of VHH

***Immunization of Ilamas with insect homogenates -*** Suspensions were mixed on basis of equal total protein concentration and aliquots were prepared, stored at -80°C and suspensions were used for immunization. Two Ilamas were immunized at weekly intervals with 6 intramuscular injections of mixed insect suspensions using Freund's Incomplete Adjuvant (FIA). Doses for immunizations were 125 µg total protein for days 0 and 6, and 62.5 µg total protein for days 13, 20, 27, and 34. At day 0 and at time of PBL collection at day 38 sera of Ilamas were collected.

***Immunization of llamas with insect midgut homogenates** -* The total protein concentration of the homogenates of *Helicoverpa armigera* and *Spodoptera exigua* is determined by Bradford using BradfordUltra reagent (Expedeon, Over Cambridgeshire, UK). A mixture of homogenate of both species is made based on equal total protein concentration. Two Ilamas, named Kuzco and Ténéré, were immunized at weekly intervals with 6 intramuscular injections of mixed Lepidoptera homogenate using Freund's Incomplete Adjuvant (FIA). Doses for immunizations were 125 µg total protein for days 0 and 6, and 63 µg total protein for days 13, 20, 27, and 34. At day 0 and at time of PBL collection at day 38 sera of *Ilamas* were collected.

***Immunization of llamas with insect midgut brush border membrane vesicles (BBMV)*** - The total protein concentration of the BBMV samples of *Helicoverpa armigera* and *Spodoptera exigua* is likewise to the homogenate samples determined by Bradford using BradfordUltra reagent. A mixture of BBMV's of both species is made based on equal total protein concentration. Two Ilamas, named Maxwell and Arpel, were immunized at weekly intervals with 6 intramuscular injections of mixed Lepidoptera BBMV's using Freund's Incomplete Adjuvant (FIA). Doses for immunizations were 125 µg total protein for days 0 and 6, and 63 µg total protein for days 13, 20, 27, and 34. At day 0 and at time of PBL collection at day 38 sera of *Ilamas* were collected.

***Immunizations of llamas with specific molecular targets** -* VHH are generated from *Ilamas* immunized with specific molecular targets. Llamas are immunized according to standard protocols with 6 boosts of VLPs displaying enriched densities of the molecular targets on their surface (Integral Molecular). Alternatively, *Ilamas* are vaccinated by DNA immunization using Nanotaxi technology (In-Cell-Art, Nantes, France) according to the manufacturer's instructions with 6 immunizations and if necessary followed by a protein boost (prime-boost strategy).

***Library construction -*** From each immunized llama a separate VHH library is made. RNA is isolated from peripheral blood lymphocytes, followed by cDNA synthesis using random hexamer primers and Superscript III according to the manufacturer's instructions (Thermo Fisher Scientific, Waltham, USA). A first PCR is performed to amplify VHH and VH DNA fragments using a forward primer mix [1:1 ratio of call001 (5'-gtcctggctgctcttctacaagg-3') and call001b b (5'-cctggctgctcttctacaaggtg-3')] and reverse primer call002 (5'-ggtacgtgctgttgaactgttcc-3'). After separation of VH and VHH DNA fragments by agarose gel electrophoresis and purification of VHH DNA fragments from gel, a second PCR is performed on VHH DNA fragments to introduce appropriate restriction sites for cloning using forward primer A6E (5'-gatgtgcagctgcaggagtctggrggagg-3') and reverse primer 38 (5'-ggactagtgcggccgctggagacggtgacctgggt-3'). The PCR fragments are digested using *Pst*I and *Eco*91I restriction enzymes (Thermo Fisher Scientific), and ligated upstream of the pIII gene in vector pMES3. The ligation products are ethanol precipitated according to standard protocols, resuspended in water, and electroporated into TG1 cells. Library sizes are at least 1E+08 independent clones for each library. Single colony PCR on randomly picked clones from the libraries is performed to assess insert percentages of the libraries. Phages from each of the libraries are produced using VCSM13 helper phage according to standard procedures.

### Example 3: Selection of VHH against

***Phage selections on coated midgut antigens*** - For selections against midgut homogenate, BBMV's and VLP's first optimal coating concentrations are determined in ELISA using an anti-actin antibody or anti-vATPase antibody. ELISA plates are coated with 10-fold serial dilutions of the antigen and incubated overnight at 4°C. The next day, plates are washed 3 times using noctuïde ringer buffer and blocked with 1% casein in noctuïde ringer buffer for 2 hours. Phages are diluted in 0.25% casein in noctuid ringer buffer and approximately 6E+11 cfu are used for each well. After binding to the well for 2 hours at room temperature, unbound phages are removed by washing the ELISA plate 15 times with noctuïde ringer buffer. To elute the bound phages, 1 mg/ml of trypsin is added to each well and the plate is incubated for 20 minutes. Eluted phages are transferred to a tube containing excess AEBSF trypsin inhibitor. The titers of the phages are compared to the titers of an unrelated control sample to assess enrichments. Phages are amplified using fresh TG1 cells according to standard procedures.

***In solution phage selections -*** Selections against biotinylated proteoliposomes are done using streptavidin beads. 10-fold serial dilutions of the antigens are mixed with phages (approximately 6x10¹¹ cfu per condition) in 0.25% casein in noctuid ringer buffer and are incubated by head-over-head rotation for 2 hours at room temperature. Streptavidin beads are prepared in 0.25% casein in noctuid ringer buffer and blocked for 30 min by head-over-head rotation. Beads and phage-antigen complexes are mixed together and incubated for 1 minute. Unbound phages are removed by washing the beads 5 times with noctuïde ringer buffer using a magnetic rack. To elute the bound phages, 1 mg/ml of trypsin is added to each condition and incubated for 20 minutes. Eluted phages are transferred to a tube containing excess AEBSF trypsin inhibitor. The titers of the phages are compared to the titers of an unrelated control sample to assess enrichments. Phages are amplified using fresh TG1 cells according to standard procedures.

***Cell-based phage selections*** - For selections on cells overexpressing a specific molecular target, per condition 10⁷ cells are mixed with phages (approximately 6x10¹¹ cfu) and incubated by head-over-head rotation for 1 hour at 4°C. After binding to the cells for 1 hour, unbound phages are removed by washing the cells 5 times. To elute the bound phages, 1 mg/ml of trypsin is added to each condition and incubated for 20 minutes. Eluted phages are transferred to a tube containing excess AEBSF trypsin inhibitor. The titers of the phages are compared to the titers of an unrelated control sample to assess enrichments. Phages are amplified using fresh TG1 cells according to standard procedures.

***Ex vivo phage selections against midgut antigens*** - For selections against *Helicoverpa armigera* and *Spodoptera exigua* midguts, L5 larvae are selected and placed on ice. The midguts are carefully removed and sliced open to make the inside available. Midguts are placed on presoaked tissue to avoid drying. Blocking is done with 1% casein in noctuid Ringer's buffer. The incubation is done for 2 hours. Afterwards, the midguts are washed one time with noctuid Ringer's buffer. Phages are suspended in noctuid Ringer's buffer and approximately 6¹¹ cfu are used for each midgut. After binding to the midguts for 2 hours, unbound phages are removed by washing the midguts 10 times with noctuid Ringer's buffer. To elute the bound phages, the midguts are placed in a tube containing 1 mg/ml of trypsin for 20 minutes. Eluted phages are transferred to a tube containing excess AEBSF trypsin inhibitor. The titers of the phages are compared to the titers of an unrelated control sample to assess enrichments. Phages are amplified using fresh TG1 cells according to standard procedures.

***In vivo phage selections against insects -*** For selections against *Helicoverpa armigera* and *Spodoptera exigua* larvae, phages are suspended in glucose water and larvae are allowed to eat ad libitum. After 24, 48 and 72 hours larvae are collected and the midguts are carefully removed and sliced open to make the inside available. Unbound phages are removed by washing the midguts 10 times with noctuid ringer buffer. To elute the bound phages, the midguts are placed in a tube containing 1 mg/ml of trypsin for 20 minutes. Eluted phages are transferred to a tube containing excess AEBSF trypsin inhibitor. The titers of the phages are compared to the titers of an unrelated control sample to assess enrichments. Phages are amplified using fresh TG1 cells according to standard procedures.
Based on the immunization strategy different selection schemes are followed (Figures 1A, 1B, 1C).

***Picking single colonies from selection outputs -*** Fresh TG1 cells are infected with serially diluted eluted phages and plated on LB agar; 2% glucose; 100 µg/ml ampicillin. Single colonies are picked in 96-well plates containing 150 µl per well 2xTY; 10% glycerol; 2% glucose; 100 µg/ml ampicillin. Plates are incubated at 37°C and stored at -80°C as master plates. From each library a total of 180 clones are picked.

### Example 4: Screening of VHH

***Single colony PCR and sequencing -*** Single colony PCR and sequencing is performed on all clones. Colonies are directly picked into a PCR premix according to the master plate layout. Single colony PCR is performed using forward primer MP57 (5'-TTATGCTTCCGGCTCGTATG-3') and reverse primer Gene III (5'-CCACAGACAGCCCTCATAG-3'). PCR products are verified on agarose gel using standard procedures. PCR products are sequenced by LGC genomics using Sanger-sequencing with primer MP57 (5'- TTATGCTTCCGGCTCGTATG-3'). Unique VHH sequences are selected for rearray.

***Rearray*, *VHH production and phage production -*** A 96-well plate containing 150 µl per well 2xTY; 10% glycerol; 2% glucose; 100 µg/ml ampicillin is inoculated with 10 µl of the TG1 stock of the good clones determined by sequencing. The plate is incubated at 37°C during the night. For production of VHH, a 96-deepwell plate containing 975 µl 2xTY; 0.1% glucose; 100 µg/ml ampicillin is inoculated with 25 µl of the overnight culture and sealed with a gas permeable seal. After growing at 37°C for 3 hours, IPTG is added to 1 mM final concentration and recombinant VHH is produced during an additional incubation for 4 hours. Cells are spun down by centrifugation at 4700 rpm for 20 minutes, supernatant is discarded and pellets are stored at -20°C overnight. Cell pellets are thawed, briefly vortexed and 120 µl 1xPBS/1M NaCl is added. Cells are resuspended by vortexing and plates are centrifuged at 4700 rpm for 20 minutes. 100 µl of the supernatant is transferred to a fresh 96-well plate. For production of phage, a 96-deepwell plate containing 250 µl 2xTY; 0.1 % glucose; 100 µg/ml ampicillin is inoculated with 10 µl of the overnight culture and sealed with a gas permeable seal. After growing at 37°C for 2 hours, 250 µl of 2xTY; 100 µg/ml ampicillin; 25 µg/ml kanamycin and helper phage (10⁹ cfu/well) is added and phages are produced during an additional incubation overnight. Cells are spun down by centrifugation at 4700 rpm for 20 minutes and 300 µl supernatant is transferred to a fresh 96-well polypropylene plate.

***Binding ELISA -*** All unique clones are tested for binding on homogenate, BBMV's or total protein extract from the midgut of *Helicoverpa armigera* and *Spodoptera exigua.* The total protein extract is made following standard procedure (KU Leuven - Animal Physiology and Neurobiology Section). The optimal coating concentration of total protein extract is determined in ELISA using an anti-actin antibody. The binding of the clones is tested as VHH and as phage. ELISA plates are coated with the antigen and incubated overnight at 4°C. The next day, plates are washed 3 times using PBS/0.05% Tween-20 and blocked with 5% skimmed milk in PBS/0.05% Tween-20 for 2 hours. Plates are washed one time with PBS/0.05% Tween-20 and a VHH premix or phage premix in an end concentration of 1% skimmed milk in PBS/0.05% Tween-20 is added and incubated for 1 hour. For each condition also a non-coated well (coated with PBS) is taken along. Non-binding VHH and phages are removed by washing the plates three times with PBS/0.05% Tween-20. Bound VHH are detected with sequential incubations with monoclonal mouse anti-histidine in 1% skimmed milk in PBS/0.05% Tween-20 and rabbit anti-mouse IgG whole molecule antibodies conjugated with alkaline phosphatase in 1% skimmed milk in PBS/0.05% Tween-20. Bound phages are detected with an anti-M13-HRP antibody in 1% skimmed milk in PBS/0.05% Tween-20. Unbound antibodies are removed by washing three times with PBS/0.05% Tween-20 and three times with PBS. For the detection of AP, pNPP in the corresponding buffer is added to the wells, while for the detection of HRP, ABTS in the corresponding buffer is added. The absorbance at 405 nm is measured and the ratio of the coated-well over the non-coated well is calculated for each clone.

***Flow cytometry -*** Flow cytometry analysis allows detection of cell surface expressed proteins or surface presented biomolecules other than proteins. Cell lines overexpressing or displaying selected molecular targets used for VHH binding assessment are the transfected grasshopper cell lines, its parental non transfected cell line, transfected *Trichoplusia ni* midgut cell line Tn-MG1 and its parental non transfected cell line. 10⁵ cells (100µl) are mixed with 1µg of VHH and detection is performed via a polyclonal rabbit anti-VHH DyLight488 or AlexaFluor488 labeled antiserum.

### Example 5: Screening for biological activity

***Production of VHH** -* VHH are produced in *E. coli* in culture flasks as epitope-tagged proteins according to standard procedures. Epitope-tagged fusion proteins are purified from the periplasmic extract with the appropriate resin, according to the manufacturer's instructions. Purified fusion proteins are concentrated and dialyzed to PBS. Alternatively fusion proteins are purified using automated purification systems using a combination of affinity and desalting columns.

***In vitro feeding assays -** H. armigera* and *S. exigua* larvae are maintained at 26±2°C, 70±5% relative humidity (RH). The artificial diet (AD) is prepared and larvae are maintained under laboratory conditions on an artificial diet and subsequent generations are used for feeding assays. Newly emerged larvae are fed on artificial diet or diet supplemented with VHH proteins. The major ingredients of the control diet are wheat germ, casein, sugar, yeast, Wesson's salt, sorbic acid, cholesterol and p-hydroxybenzoic acid. VHH diet is prepared by incorporating VHH into the artificial diet. Binding capability of VHH after preparation of diet is carried out after by feeding larvae with the artificial diet supplemented with fluorophore labeled VHH and verification of binding through confocal microscopy on isolated midgut. The VHH denatured by heating was used as alternative control. Thirty neonates are transferred to control as well as test diets. Three sets containing thirty neonates each are maintained on respective diet as replicates (i.e. total 90 insects each on control and test diet and the assay is repeated three times). Fresh diets are fed daily in individual vials containing one larva. Insects are maintained at 26±2°C, 70% RH. The feeding assays are continued up to 15 d and the larval masses are recorded.

***Re-cloning, production & purification, feeding assays after spray-on application*** - Coding sequence of VHH is re-cloned in an expression vector dedicated for expression in *P. pastoris* derived from pPpT4-αS which contains the AOX1 promoter, a resistance gene for zeocin, a multicloning site and the α-factor signal sequence for secreted expression. The vector is linearized with Swal before integrative transformation. VHH proteins are expressed in *P. pastoris* CBS7435 cells in culture. Expression is induced by addition of MeOH to 1%. After spinning the cell cultures, the supernatant is further clarified and filtered. Purity and integrity of all VHH proteins are verified by SDS-PAGE. The efficacy of the VHH with potent *in vitro* insecticidal activity against *H. armigera* and *S. exigua* is further evaluated *in planta* via feeding bio-assays on (i) detached leaves from tomato and lettuce plants and (ii) on greenhouse-grown tomato and lettuce plants. Detached leaves or whole plants are sprayed in a spraying cabinet with an aqueous solution. After spraying, the spray deposit is allowed to dry and leaves or plants are inoculated with newly hatched larvae (<10 h old). Each larva is transferred using a moistened soft paintbrush to the appropriate detached leaves or whole plants. For each assay, 5 or 10 larvae are placed per leaf or plant and the experiment was monitored by assessing leaf damage and larva growth weight in time.

### Example 6: Experimental set-up for design and screening of VHH-toxin fusion constructs

***Cloning of fusion constructs*** - The coding sequences of VHH proven to bind to midgut antigen (either complex antigen such as midgut homogenate, BBMVs or specific molecular targets known to be displayed on the midgut) are cloned in-frame with the coding sequence of the toxin with a linker sequence in between, in an expression vector derived from pUC119 which contains the LacZ promoter, a resistance gene for kanamycin, a multicloning site and the OmpA signal peptide sequence. In frame with the VHH-toxin coding sequence, the vector codes for a C-terminal epitope tag. Alternatively, sequences are cloned in frame with pIII in a phagemid vector derived from pUC119, or in a vector dedicated for expression in *P. pastoris* derived from pPpT4-αS as described above.

***VHH-toxin fusion protein characterization -*** For verification of the binding capacity of the VHH in a fusion protein context, VHH-toxin fusion proteins are expressed in *E. coli* TG-1 cells as epitope-tagged proteins in culture. Expression is induced by addition of 1 mM IPTG and is allowed to continue for 3 hours at 37°C. After spinning the cell cultures, periplasmic extracts are prepared by freeze-thawing the pellets and resuspension in dPBS. These extracts are used as starting material for affinity chromatography. VHH-toxin fusion proteins are eluted from the column, concentrated and subsequently desalted towards PBS. Purity and integrity of all VHH-toxin fusion proteins are verified by polyacrylamide gel electrophoresis (SDS-PAGE), while the presence of tags was verified by western blotting using anti-epitope antibodies for detection. Binding of the fusion proteins is then verified using the same assay as the primary binding assay from which the VHH-portion was selected.

***In vitro feeding assays*** - *H. armigera* and *S. exigua* larvae are maintained at 26±2°C, 70±5% relative humidity (RH). The artificial diet (AD) is prepared and larvae are maintained under laboratory conditions on an artificial diet and subsequent generations are used for feeding assays. Newly emerged larvae are fed on artificial diet or diet supplemented with VHH-toxin proteins. The major ingredients of the control diet are wheat germ, casein, sugar, yeast, Wesson's salt, sorbic acid, cholesterol and p-hydroxybenzoic acid. VHH-toxin diet is prepared by incorporating VHH-toxin into the artificial diet. Binding capability of VHH-toxin after preparation of diet is carried out after by feeding larvae with the artificial diet supplemented with fluorophore labeled VHH-toxin and verification of binding through confocal microscopy on isolated midgut. The VHH-toxin denatured by heating was used as alternative control. Thirty neonates are transferred to control as well as test diets. Three sets containing thirty neonates each are maintained on respective diet as replicates (i.e. total 90 insects each on control and test diet and the assay is repeated three times). Fresh diets are fed daily in individual vials containing one larva. Insects are maintained at 26±2°C, 70% RH. The feeding assays are continued up to 15 d and the larval masses are recorded.

***Re-cloning, production* & *purification, feeding assays after spray-on application -*** Coding sequence of VHH-toxin is re-cloned in an expression vector dedicated for expression in *P. pastoris* derived from pPpT4-αS which contains the AOX1 promoter, a resistance gene for zeocin, a multicloning site and the α-factor signal sequence for secreted expression. The vector is linearized with Swal before integrative transformation. VHH-toxin fusion proteins are expressed in *P. pastoris* CBS7435 cells in culture. Expression is induced by addition of MeOH to 1%. After spinning the cell cultures, the supernatant is further clarified and filtered. Purity and integrity of all VHH-toxin fusion proteins are verified by SDS-PAGE. The efficacy of the VHH-toxin with potent *in vitro* insecticidal activity against *H. armigera* and S. *exigua* is further evaluated *in planta* via feeding bio-assays on (i) detached leaves from tomato and lettuce plants and (ii) on greenhouse-grown tomato and lettuce plants. Detached leaves or whole plants are sprayed in a spraying cabinet with an aqueous solution. After spraying, the spray deposit is allowed to dry and leaves or plants are inoculated with newly hatched larvae (<10 h old). Each larva is transferred using a moistened soft paintbrush to the appropriate detached leaves or whole plants. For each assay, 5 or 10 larvae are placed per leaf or plant and the experiment was monitored by assessing leaf damage and larva growth weight in time.

### References

Alger, N.E., and E.J. Cabrera. 1972. An increase in death rate of Anopheles stephensi fed on rabbits immunized with mosquito antigen. Journal of economic entomology 65:165-168.
Altschul, S.F., W. Gish, W. Miller, E.W. Myers, and D.J. Lipman. 1990. Basic local alignment search tool. J Mol Biol 215:403-410.
Carrière, Y., J.A. Fabrick, and B.E. Tabashnik. 2016. Can Pyramids and Seed Mixtures Delay Resistance to Bt Crops? Trends Biotechnol 34:291-302.
Casu, R., C. Eisemann, R. Pearson, G. Riding, I. East, A. Donaldson, L. Cadogan, and R. Tellam. 1997. Antibody-mediated inhibition of the growth of larvae from an insect causing cutaneous myiasis in a mammalian host. Proc Natl Acad Sci U S A 94:8939-8944.
Crickmore, N., D.R. Zeigler, J. Feitelson, E. Schnepf, J. Van Rie, D. Lereclus, J. Baum, and D.H. Dean. 1998. Revision of the nomenclature for the Bacillus thuringiensis pesticidal crystal proteins. Microbiol Mol Biol Rev 62:807-813.
Dal Peraro, M., and F.G. van der Goot. 2016. Pore-forming toxins: ancient, but never really out of fashion. Nat Rev Microbiol 14:77-92.
Dimitrov, D.S. 2009. Engineered CH2 domains (nanoantibodies). MAbs 1:26-28.
Ghosh, K.N., and J. Mukhopadhyay. 1998. The effect of anti-sandfly saliva antibodies on Phlebotomus argentipes and Leishmania donovani. Int J Parasitol 28:275-281.
Henikoff, S., and J.G. Henikoff. 1992. Amino acid substitution matrices from protein blocks. Proc Natl Acad Sci U S A 89:10915-10919.
Meyers, J.I., M. Gray, and B.D. Foy. 2015. Mosquitocidal properties of IgG targeting the glutamate-gated chloride channel in three mosquito disease vectors (Diptera: Culicidae). J Exp Biol 218:1487-1495.
Nuttall, S.D., U.V. Krishnan, L. Doughty, K. Pearson, M.T. Ryan, N.J. Hoogenraad, M. Hattarki, J.A. Carmichael, R.A. Irving, and P.J. Hudson. 2003. Isolation and characterization of an IgNAR variable domain specific for the human mitochondrial translocase receptor Tom70. Eur J Biochem 270:3543-3554.
Schlein, Y., and T.C. Lewis. 1976. Lesions in haematophagous flies after feeding on rabbits immunized with fly tissues. Physiological Entomology 1:
Sutherland, G.B., and A.B. Ewen. 1974. Fecundity decrease in mosquitoes ingesting blood from specifically sensitized mammals. J Insect Physiol 20:655-660.
Thompson, J.D., D.G. Higgins, and T.J. Gibson. 1994. CLUSTAL W: improving the sensitivity of progressive multiple sequence alignment through sequence weighting, position-specific gap penalties and weight matrix choice. Nucleic Acids Res 22:4673-4680.
Tramontano, A., E. Bianchi, S. Venturini, F. Martin, A. Pessi, and M. Sollazzo. 1994. The making of the minibody: an engineered beta-protein for the display of conformationally constrained peptides. J Mol Recognit 7:9-24.
van Frankenhuyzen, K. 2009. Insecticidal activity of Bacillus thuringiensis crystal proteins. J Invertebr Pathol 101:1-16.
Wesolowski, J., V. Alzogaray, J. Reyelt, M. Unger, K. Juarez, M. Urrutia, A. Cauerhff, W. Danquah, B. Rissiek, F. Scheuplein, N. Schwarz, S. Adriouch, O. Boyer, M. Seman, A. Licea, D.V. Serreze, F.A. Goldbaum, F. Haag, and F. Koch-Nolte. 2009. Single domain antibodies: promising experimental and therapeutic tools in infection and immunity. Med Microbiol Immunol 198:157-174.
Wolfersberger, M.G., P. Lüthy, A. Maurer, P. Parenti, V.F. Sacchi, B. Giordana, and G.M. Hanozet. 1987. Preparation and partial characterization of amino acid transporting brush border membrane vesicles from the larval midgut of the cabbage butterfly (Pieris brassicae). Comparative Biochemistry and Physiology Part A: Physiology 86:301-308.

## Claims

1. An insect-controlling polypeptide comprising a binding domain capable of binding a binding site of an insect, wherein said binding site is in the digestive tract of said insect, and wherein said binding domain is an antigen-binding domain.

2. The insect-controlling polypeptide of claim 1, wherein said antigen-binding domain is selected from the group consisting of VHHs and alphabodies, and functional fragments thereof.

3. The insect-controlling polypeptide of claim 1 or 2, wherein said binding site is in the midgut and/or hindgut of said insect.

4. The insect-controlling polypeptide of any of claims 1-3, wherein said binding site is selected from the group consisting of CAATCH1, KAAT1, V-ATPase subunit a, V-ATPase subunit c, V-ATPase subunit e, IIM14, IIM21, ABCH1, SSK, MESH, K+/2H+ antiporter, CI- channel, and Cl-/HCO3-antiporter.

5. The insect-controlling polypeptide of any of claims 1-4, further comprising a toxin moiety.

6. A nucleic acid comprising a polynucleotide encoding an insect-controlling polypeptide according to any of claims 1-5.

7. A nucleic acid construct comprising the nucleic acid of claim 6.

8. A host cell comprising the polypeptide of any of claims 1-5, the nucleic acid of claim 6, and/or the nucleic acid construct according to claim 7.

9. A composition for preventing insect infestation and/or for controlling insects comprising at least one polypeptide according to any of claims 1-5, a nucleic acid of claim 6, a nucleic acid construct according to claim 7, and/or a host cell of claim 8.

10. Use of a polypeptide according to any of claims 1-5, a nucleic acid of claim 6, a nucleic acid construct according to claim 7, a host cell of claim 8, and/or a composition of claim 9 for preventing infestation by said insect or controlling said insect.

11. A method for controlling insects, comprising the step of contacting or feeding said insects with at least one polypeptide according to any of claims 1-5, a nucleic acid of claim 6, a nucleic acid construct according to claim 7, a host cell of claim 8, and/or a composition of claim 9.

12. A plant or plant part treated with and comprising at least one polypeptide according to any of claims 1-5, a nucleic acid of claim 6, a nucleic acid construct according to claim 7, a host cell of claim 8, and/or a composition of claim 9.

13. A transgenic plant cell comprising a polypeptide according to any of claims 1-5, a nucleic acid of claim 6, and/or a nucleic acid construct according to claim 7.

14. A transgenic plant comprising a polypeptide according to any of claims 1-5, a nucleic acid of claim 6, and/or a nucleic acid construct according to claim 7.

15. A seed of the transgenic plant according to claim 14, wherein said seed comprises a polypeptide according to any of claims 1-5, a nucleic acid of claim 6, and/or a nucleic acid construct according to claim 7.

16. A commodity product produced from the plant or plant part according to claim 12, the transgenic plant cell of claim 13, the transgenic plant according to claim 14 or the seed of claim 15, wherein said commodity product comprises the polypeptide of any of claims 1-5.
